(19) Europäisches Patentamt
European Patent Office
Office européen des brevets

(11) **EP 2 172 263 A1**

(12) **EUROPEAN PATENT APPLICATION**
published in accordance with Art. 153(4) EPC

(43) Date of publication:
**07.04.2010 Bulletin 2010/14**

(21) Application number: **08790744.0**

(22) Date of filing: **30.06.2008**

(51) Int Cl.:
*B01J 13/00* (2006.01)　　*A61K 8/34* (2006.01)
*A61K 8/37* (2006.01)　　*A61K 8/60* (2006.01)
*A61K 8/84* (2006.01)　　*A61Q 19/00* (2006.01)

(86) International application number:
**PCT/JP2008/061831**

(87) International publication number:
**WO 2009/005032 (08.01.2009 Gazette 2009/02)**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **02.07.2007 JP 2007174456**

(71) Applicant: **The Nisshin OilliO Group, Ltd.
Tokyo 104-8285 (JP)**

(72) Inventors:
• **KACHI, Hisanori
Yokohama-shi
Kanagawa 235-8558 (JP)**

• **NAKAMURA, Kayo
Yokohama-shi
Kanagawa 235-8558 (JP)**
• **MATSUZAWA, Makoto
Yokohama-shi
Kanagawa 235-8558 (JP)**

(74) Representative: **Vuillermoz, Bruno et al
Cabinet Laurent & Charras
"Le Contemporain"
50, Chemin de la Bruyère
69574 Dardilly Cédex (FR)**

(54) **W/O/W EMULSION COMPOSITION**

(57)　A W/O/W emulsion composition having high emulsion stability, which can be prepared extremely easily and for which the production efficiency of the W/O/W emulsion product is favorable, and a method of producing such a W/O/W emulsion composition. The W/O/W emulsion composition of the present invention includes 0.0011 to 60% by mass of a component (A) described below and 0.001 to 10% by mass of a component (B) described below; wherein, a mass ratio between the component (A) and the component (B) is within a range from 1:0.01 to 1:1.4. Component (A): a sugar fatty acid ester composition obtained by esterifying a sugar and a fatty acid of 8 to 28 carbon atoms, in which a hydroxyl value of said sugar fatty acid ester composition is within a range from 20 to 220. Component (B): a nonionic surfactant having an HLB value of not less than 7.

EP 2 172 263 A1

**Description**

BACKGROUND OF THE INVENTION

Field of the Invention

The present invention relates to a W/O/W emulsion composition (water-in-oil-in-water emulsion composition) that can be prepared extremely easily and exhibits excellent emulsion stability over a long period of time.
Priority is claimed on Japanese Patent Application No. 2007-174456, filed July 2, 2007, the content of which is incorporated herein by reference.

Description of the Related Art

W/O/W emulsion compositions are composite emulsion compositions containing a water-in-oil emulsion dispersed within an aqueous component. By encapsulating any of a variety of useful components within the inner water phase of the W/O/W emulsion composition, these W/O/W emulsion compositions are able to be used as functional emulsion compositions having effects such as a sustained release action or an antioxidant action, and as such, are extremely useful as medications, cosmetic products and foodstuffs and the like.
However, a problem arises in that the emulsion stability of conventional W/O/W emulsion compositions tends to be very poor.
Methods that have been reported for improving the emulsion stability of W/O/W emulsion compositions include methods that use a polymer to stabilize the composition (see Patent Documents 1 and 2) and a method that uses a specific emulsifier to stabilize the composition (see Patent Document 3).
However, in those cases where a W/O/W emulsion composition having superior emulsion stability is required, none of the W/O/W emulsion compositions obtained using the above methods exhibits a satisfactory level of emulsion stability.
Furthermore, in most methods of preparing W/O/W emulsion compositions, the emulsification is generally divided into two stages. Namely, in the first stage, an inner water phase is dispersed within an oil phase to prepare a W/O emulsion, and in the second stage, this W/O emulsion is dispersed within an outer water phase to prepare the W/O/W emulsion composition.
However, in an industrial setting, this method of preparation requires that separate tanks are used for the emulsification tank used in preparing the W/O emulsion and the emulsification tank used in preparing the final W/O/W emulsion composition. As a result, steps for heating, dispersing, cooling and transferring the W/O emulsion must be included, which makes the method unfavorably complex.
Furthermore, it is already known from experience that when an O/W emulsion is prepared using a one-step emulsification method, W/O/W emulsion products may be coincidentally and partially generated, but these W/O/W emulsion products suffer from poor production efficiency and poor stability, meaning they are unable to be used in practical applications.
As a result of the circumstances described above, the development of a W/O/W emulsion composition which has a high level of emulsion stability, can be prepared extremely easily, and exhibits favorable production efficiency of the W/O/W emulsion product has been keenly sought.

[Patent Document 1]
Japanese Unexamined Patent Application, First Publication No. Hei 11-33391
[Patent Document 2]
Japanese Laid-Open Patent Application No. 2004-307414
[Patent Document 3]
Japanese Laid-Open Patent Application No. 2005-132794

SUMMARY OF THE INVENTION

An object of the present invention is to provide a W/O/W emulsion composition having high emulsion stability, which can be prepared extremely easily and for which the production efficiency of the W/O/W emulsion product is favorable, and also to provide a method of producing such a W/O/W emulsion composition.
As a result of intensive investigation aimed at achieving the above object, the inventors of the present invention discovered that by blending a specific sugar fatty acid ester composition and a nonionic surfactant having an HLB value of not less than 7 in a specific blend ratio, a W/O/W emulsion composition could be obtained that exhibited superior emulsion stability, was extremely easy to prepare, and exhibited favorable production efficiency of the W/O/W emulsion product, and they were therefore able to complete the present invention.

In other words, a first aspect of the present invention is a W/O/W emulsion composition that includes 0.001 to 60% by mass of a component (A) described below and 0.001 to 10% by mass of a component (B) described below, wherein the mass ratio between the component (A) and the component (B) is within a range from 1:0.01 to 1:1.4.

Component (A): a sugar fatty acid ester composition obtained by esterifying a sugar and a fatty acid of 8 to 28 carbon atoms, wherein the hydroxyl value of the sugar fatty acid ester composition is within a range from 20 to 220.

Component (B): a nonionic surfactant having an HLB value of not less than 7.

A second aspect of the present invention is a W/O/W emulsion composition according to the first aspect, wherein a constituent sugar of the sugar fatty acid ester composition of the component (A) is one or more sugars selected from the group consisting of inositol, trehalose, lactitol, maltitol, raffinose, mannitol, xylitol and erythritol.

A third aspect of the present invention is a W/O/W emulsion composition according to the first aspect, wherein said constituent sugar of the sugar fatty acid ester composition of the component (A) is trehalose.

A fourth aspect of the present invention is a W/O/W emulsion composition according to any one of the first to third aspects described above, wherein the constituent fatty acid of 8 to 28 carbon atoms of the sugar fatty acid ester composition of the component (A) is one or more compounds selected from the group consisting of octanoic acid, decanoic acid, lauric acid, myristic acid, palmitic acid, stearic acid, arachidonic acid, palmitoleic acid, oleic acid, linoleic acid, linolenic acid, behenic acid, erucic acid, 2-ethylhexanoic acid, 2-hexyldecanoic acid, 2-heptylundecylenic acid, isostearic acid, 2-octyldodecanoic acid and 2-dodecyltetradecanoic acid.

A fifth aspect of the present invention is a W/O/W emulsion composition according to any one of the first to third aspects, wherein the constituent fatty acid of 8 to 28 carbon atoms of the sugar fatty acid ester composition of the component (A) is isostearic acid.

A sixth aspect of the present invention is a W/O/W emulsion composition according to any one of the first to fifth aspects described above, wherein the component (B) is a nonionic surfactant having an HLB value of not less than 7 that contains a polyoxyethylene group.

A seventh aspect of the present invention is a W/O/W emulsion composition according to any one of the first to sixth aspects, further including 0.1 to 45% by mass of a polyhydric alcohol as a component (C).

An eighth aspect of the present invention is a W/O/W emulsion composition according to the seventh aspect, wherein the component (C) is one or more polyhydric alcohols selected from the group consisting of glycerol, diglycerol, triglycerol, polyethylene glycol, propylene glycol, 1,3-butylene glycol, 3-methyl-1,3-butanediol, 1,2-pentanediol, 1,2-hexanediol and hexylene glycol.

A ninth aspect of the present invention is a W/O/W emulsion composition according to any one of the first to eighth aspects described above, wherein the W/O/W emulsion composition is a cosmetic preparation.

A tenth aspect of the present invention is a W/O/W emulsion composition according to the ninth aspect, wherein the cosmetic preparation is milky lotions, a cream, an essence, a lotion, an ointment or a pack.

An eleventh aspect of the present invention is a cosmetic preparation containing a W/O/W emulsion composition according to any one of the first to eighth aspects described above.

A twelfth aspect of the present invention is a cosmetic preparation according to the eleventh aspect, wherein the cosmetic preparation is milky lotions, a cream, an essence, a lotion, an ointment or a pack.

A thirteenth aspect of the present invention is a method of producing a W/O/W emulsion composition, the method including: adding a water phase containing water to an oil phase, and performing a phase inversion emulsification, wherein the oil phase comprises 0.001 to 60% by mass of a component (A) described below and 0.001 to 10% by mass of a component (B) described below, and a mass ratio between the component (A) and the component (B) is within a range from 1:0.01 to 1:1.4.

Component (A): a sugar fatty acid ester composition obtained by esterifying a sugar and a fatty acid of 8 to 28 carbon atoms, in which a hydroxyl value of said sugar fatty acid ester composition is within a range from 20 to 220.

Component (B): a nonionic surfactant having an HLB value of not less than 7.

The present invention is able to provide a W/O/W emulsion composition which can be prepared by a simple phase inversion emulsification method that has recently become widespread, and which exhibits a high level of emulsion stability and a favorable level of production efficiency for the W/O/W emulsion product.

DETAILED DESCRIPTION OF THE INVENTION

A more detailed description of the present invention is presented below.

A W/O/W emulsion composition of the present invention includes 0.001 to 60% by mass of a component (A) described below and 0.001 to 10% by mass of a component (B) described below, wherein the mass ratio between the component (A) and the component (B) is within a range from 1:0.01 to 1:1.4.

Component (A): a sugar fatty acid ester composition obtained by esterifying a sugar and a fatty acid of 8 to 28 carbon atoms, wherein the hydroxyl value of the sugar fatty acid ester composition is within a range from 20 to 220.

Component (B): a nonionic surfactant having an HLB value of not less than 7.

The W/O/W emulsion composition of the present invention contains a sugar fatty acid ester composition obtained by esterifying a sugar and a fatty acid of 8 to 28 carbon atoms. Including the sugar fatty acid ester composition obtained by esterifying a sugar and a fatty acid of 8 to 28 carbon atoms enables the preparation of a highly stable W/O/W emulsion composition for which the preparation is extremely easy and the production efficiency of the W/O/W product is favorable. In the following description, the sugar fatty acid ester composition obtained by esterifying a sugar and a fatty acid of 8 to 28 carbon atoms is termed "component (A)".

Examples of the sugar used in generating the sugar fatty acid ester composition of the component (A) include monosaccharides, oligosaccharides and sugar alcohols. Of these, inositol, trehalose, lactitol, maltitol, raffinose, mannitol, xylitol and erythritol are preferred, inositol, trehalose, lactitol, maltitol and raffinose are more preferred, and trehalose is the most desirable. These sugars may be used individually or in combinations containing two or more different sugars. By using one or more of the above sugars as the sugar for generating the sugar fatty acid ester composition of the component (A), a sugar fatty acid ester composition is obtained that enables the production of a highly stable W/O/W emulsion composition for which the preparation is extremely easy and the production efficiency of the W/O/W product is favorable.

Examples of the fatty acid of 8 to 28 carbon atoms used in generating the sugar fatty acid ester composition of the component (A) include linear saturated fatty acids such as octanoic acid, decanoic acid, lauric acid, myristic acid, palmitic acid, stearic acid, behenic acid and arachidonic acid, linear unsaturated fatty acids such as palmitoleic acid, oleic acid, linoleic acid, linolenic acid and erucic acid, and branched saturated fatty acids such as 2-ethylhexanoic acid, hexylundecylenic acid, isostearic acid, methyl-branched isostearic acid, 2-octyldodecanoic acid and 2-dodecyltetradecanoic acid. Of these, octanoic acid, decanoic acid, lauric acid, myristic acid, palmitic acid, stearic acid, arachidonic acid, palmitoleic acid, oleic acid, linoleic acid, linolenic acid, behenic acid, erucic acid, 2-ethylhexanoic acid, 2-hexyldecanoic acid, 2-heptylundecylenic acid, isostearic acid, 2-octyldodecanoic acid and 2-dodecyltetradecanoic acid are preferred, and palmitic acid, stearic acid, behenic acid, oleic acid and isostearic acid are the most desirable. These fatty acids of 8 to 28 carbon atoms may be used individually or in combinations containing two or more different compounds. By using one or more of the above compounds as the fatty acid of 8 to 28 carbon atoms used in generating the sugar ester composition of the component (A), a sugar fatty acid ester composition is obtained that enables the production of a highly stable W/O/W emulsion composition for which the preparation is extremely easy and the production efficiency of the W/O/W product is favorable.

The hydroxyl value of the sugar fatty acid ester composition of the component (A) is preferably within a range from 20 to 220, is more preferably from 20 to 200, and is most preferably from 25 to 180. The hydroxyl value refers to the value obtained using the hydroxyl value measurement method disclosed in the general test methods in the Japanese standards of quasi-drug ingredients. Provided the hydroxyl value of the sugar fatty acid ester composition of the component (A) satisfies the above range, a highly stable W/O/W emulsion composition can be obtained for which the preparation is extremely easy and the production efficiency of the W/O/W product is favorable.

The degree of esterification of each of the different esters within the sugar fatty acid ester composition of the component (A) can be expressed in terms of an esterification rate. The esterification rate is dependent on the number of hydroxyl groups within the sugar that is used, and can be calculated using the formula below.

$$(\text{Esterification rate } (\%)) = (\text{Number of ester groups within the sugar fatty acid}$$

$$\text{ester composition}) / (\text{number of hydroxyl groups within the sugar used in forming the}$$

$$\text{sugar fatty acid ester composition prior to esterification}) \times 100$$

For example, in the case where trehalose, which contains 8 hydroxyl groups, is used as the sugar, and 5 of those 8 hydroxyl groups are esterified to form a trehalose pentaester, the esterification rate is calculated as follows.

$$(\text{Esterification rate}) = 5/8 \times 100 = 62.5\%$$

The esterification rates for each of the esters of trehalose which contains 8 hydroxyl groups, lactitol which contains 9 hydroxyl groups, and raffinose which contains 11 hydroxyl groups are listed below in Tables 1 to 3.

[Table 1]

| Table 1: Esterification rates of each of the esters of trehalose (number of hydroxyl groups: 8) | | |
|---|---|---|
| Type of ester | Number of ester groups | Esterification rate (%) |
| Monoester | 1 | 12.5 |
| Diester | 2 | 25.0 |
| Triester | 3 | 37.5 |
| Tetraester | 4 | 50.0 |
| Pentaester | 5 | 62.5 |
| Hexaester | 6 | 75.0 |
| Heptaester | 7 | 87.5 |
| Octaester | 8 | 100 |

[Table 2]

| Table 2: Esterification rates of each of the esters of lactitol (number of hydroxyl groups: 9) | | |
|---|---|---|
| Type of ester | Number of ester groups | Esterification rate (%) |
| Monoester | 1 | 11.1 |
| Diester | 2 | 22.2 |
| Triester | 3 | 33.3 |
| Tetraester | 4 | 44.4 |
| Pentaester | 5 | 55.6 |
| Hexaester | 6 | 66.7 |
| Heptaester | 7 | 77.8 |
| Octaester | 8 | 88.9 |
| Nonaester | 9 | 100 |

[Table 3]

| Table 3: Esterification rates of each of the esters of raffinose (number of hydroxyl groups: 11) | | |
|---|---|---|
| Type of ester | Number of ester groups | Esterification rate (%) |
| Monoester | 1 | 9.1 |
| Diester | 2 | 18.2 |
| Triester | 3 | 27.3 |
| Tetraester | 4 | 36.4 |
| Pentaester | 5 | 45.5 |
| Hexaester | 6 | 54.5 |
| Heptaester | 7 | 63.6 |
| Octaester | 8 | 72.7 |
| Nonaester | 9 | 81.8 |

(continued)

| Table 3: Esterification rates of each of the esters of raffinose (number of hydroxyl groups: 11) | | |
| --- | --- | --- |
| Type of ester | Number of ester groups | Esterification rate (%) |
| Decaester | 10 | 90.9 |
| Undecaester | 11 | 100 |

In the sugar fatty acid ester composition of the component (A), the total amount of esters for which the esterification rate is at least 40.0% but less than 90.0% is preferably within a range from 40 to 100%, more preferably from 50 to 95%, and most preferably from 60 to 90%. Further, in the sugar fatty acid ester composition of the component (A), the total amount of esters for which the esterification rate is less than 40.0% is preferably within a range from 0 to 40%, more preferably from 0 to 35%, and most preferably from 0 to 30%. In this description, the amount of each ester refers to the area percentage (%) of the peak area corresponding with that ester, which is obtained by high-performance liquid chromatography analysis (hereafter abbreviated as HPLC) using the measurement conditions and calculation methods described below. The total amount of esters can be calculated by totaling the area percentages (%) for the peak areas of each of the different esters obtained by HPLC.

Specifically, in the case of a trehalose fatty acid ester composition obtained by esterifying trehalose, which contains 8 hydroxyl groups, the total amount of esters for which the esterification rate is at least 40.0% but less than 90.0% refers to the combined amounts of the tetraester, pentaester, hexaester and heptaester, whereas the total amount of esters for which the esterification rate is less than 40.0% refers to the combined amounts of the monoester, diester and triester. In the case of a lactitol fatty acid ester composition obtained by esterifying lactitol, which contains 9 hydroxyl groups, the total amount of esters for which the esterification rate is at least 40.0% but less than 90.0% refers to the combined amounts of the tetraester, pentaester, hexaester, heptaester and octaester, whereas the total amount of esters for which the esterification rate is less than 40.0% refers to the combined amounts of the monoester, diester and triester. Furthermore, in the case of a raffinose fatty acid ester composition obtained by esterifying raffinose, which contains 11 hydroxyl groups, the total amount of esters for which the esterification rate is at least 40.0% but less than 90.0% refers to the combined amounts of the pentaester, hexaester, heptaester, octaester and nonaester, whereas the total amount of esters for which the esterification rate is less than 40.0% refers to the combined amounts of the monoester, diester, triester and tetraester.

If the total amount of esters for which the esterification rate is at least 40.0% but less than 90.0% within the sugar fatty acid ester composition of the component (A), and the total amount of esters for which the esterification rate is less than 40.0% within the sugar fatty acid ester composition of the component (A) satisfy the above ranges, then a highly stable W/O/W emulsion composition can be obtained for which the preparation is extremely easy and the production efficiency of the W/O/W product is favorable.

As described above, the total amount of esters for which the esterification rate is at least 40.0% but less than 90.0% within the sugar fatty acid ester composition of the component (A), and the total amount of esters for which the esterification rate is less than 40.0% within the sugar fatty acid ester composition of the component (A) can be calculated by totaling the area percentages (%) for the peak areas of each of the different esters obtained by HPLC. The HPLC analysis can be performed using a differential refractive index (RI) method that combines a GPC column and an ODS column, with reference to "Determination of Sucrose Fatty Acid Ester by High-performance Liquid Chromatography", J. Oleo Sci., Vol. 50, No. 4 (2001).

Analysis using a GPC column enables the separation of residual raw materials, and the monoester, diester, triester and polyesters from the sugar fatty acid ester composition. The GPC column does not enable separation of esters containing 4 or more ester groups, such as the tetraester, pentaester, hexaester, heptaester, octaester, nonaester, decaester and undecaester, and these esters containing 4 or more ester groups are measured as a mixture (of polyesters).

Further, analysis using an ODS column enables the separation of esters containing 4 or more ester groups, such as the tetraester, pentaester, hexaester, heptaester, octaester, nonaester, decaester and undecaester. In the analysis using an ODS column, the peaks associated with residual raw materials and the monoester, diester and triester overlap with the noise peak derived from the solvent medium, meaning accurate separation of these components is impossible.

Accordingly, by combining the measurement results from a GPC column and an ODS column, an area percentage (%) can be calculated for the peak area associated with each ester.

Details relating to the measurement conditions and calculation methods are described below.

[HPLC Measurement Conditions]

The HPLC measurement conditions (measurement conditions A) employed when using a GPC column to calculate area % values for the monoester, diester, triester and polyesters within the sugar fatty acid ester composition of the component (A) are described below. In this description, the term "polyesters" refers to the combination of esters containing 4 or more ester groups (such as the tetraester, pentaester, hexaester, heptaester, octaester, nonaester, decaester and undecaester) (this definition also applies in subsequent descriptions).

Measurement conditions A

Column: four series-connected GPC columns of internal diameter 7.8 mm and length 300 mm packed with a 5 $\mu$m styrenedivinylbenzene substrate

Mobile phase: tetrahydrofuran

Column temperature: 40°C

Mobile phase flow rate: 0.5 mL/min

Detection: differential refractive index (RI)

The HPLC measurement conditions (measurement conditions B) employed when using an ODS column to calculate the proportion of each of the esters within the polyester fraction of the sugar fatty acid ester composition of the component (A) are described below.

Measurement conditions B

Column: an ODS column of internal diameter 4.6 mm and length 150 mm packed with a 5 $\mu$m substrate

Mobile phase: tetrahydrofuran : methanol = 55:45 (volume ratio)

Column temperature: 40°C

Mobile phase flow rate: 0.8 mL/min

Detection: differential refractive index (RI)

[Method of Calculating Area Percentage (%) for each Ester]

The method used for calculating the area percentages (%) for the monoester, diester and triester (calculation method (1)) is described below.

The peak areas for the residual raw material, the monoester, the diester and the triester, each calculated as a percentage relative to the total peak area obtained when HPLC analysis is performed using the GPC columns described in the measurement conditions A, are recorded as the area percentages (%) for each ester.

The method used for calculating the area percentage (%) for the polyesters (calculation method (2)) is described below.

The total peak area for all peaks other than the residual raw material, the monoester, the diester and the triester, calculated as a percentage (X) relative to the total peak area obtained when HPLC analysis is performed using the GPC columns described in the measurement conditions A, is recorded as the polyester area percentage (%).

The method used for calculating the proportion of each of the esters containing 4 or more ester groups within the polyester fraction (calculation method (3)) is described below.

The combined total of the peak areas for each of the esters containing 4 or more ester groups obtained when HPLC analysis is performed using the ODS column described in the measurement conditions B is deemed (Y), and the peak areas for each of the esters containing 4 or more ester groups relative to (Y) are recorded as the proportions for each of the esters containing 4 or more ester groups within the polyester fraction.

The method used for calculating the area percentage (%) for each of the esters containing 4 or more ester groups (calculation method (4)) is described below.

The polyester area percentage (%) (X) calculated using the above calculation method (2) is multiplied by each of the peak area proportions for the esters containing 4 or more ester groups within the polyester fraction calculated using the above calculation method (3), thus yielding area percentages (%) for each ester containing 4 or more ester groups.

For example, when HPLC measurement using an ODS column is performed in accordance with the measurement conditions B, if the peak area of the tetraester is termed (a), the peak area of the pentaester is termed (b), the peak area of the hexaester is termed (c), the peak area of the heptaester is termed (d), the peak area of the octaester is termed (e), the peak area of the nonaester is termed (f), the peak area of the decaester is termed (g), and the peak area of the undecaester is termed (h), then the area percentages (%) for each of these esters are calculated using the equations shown below.

$$\text{Tetraester area percentage (\%)} = (X) \times (a) \, / \, (Y)$$

$$\text{Pentaester area percentage (\%)} = (X) \times (b) \, / \, (Y)$$

$$\text{Hexaester area percentage (\%)} = (X) \times (c) / (Y)$$

$$\text{Heptaester area percentage (\%)} = (X) \times (d) / (Y)$$

$$\text{Octaester area percentage (\%)} = (X) \times (e) / (Y)$$

$$\text{Nonaester area percentage (\%)} = (X) \times (f) / (Y)$$

$$\text{Decaester area percentage (\%)} = (X) \times (g) / (Y)$$

$$\text{Undecaester area percentage (\%)} = (X) \times (h) / (Y)$$

The method used for calculating the total amount of esters (calculation method (5)) is as follows.
The area percentage (%) obtained by totaling the area percentages (%) for each of the esters calculated using the above calculation method (1) and the above calculation method (4) is recorded as the total amount of esters.
For example, in the case of a trehalose fatty acid ester composition obtained by esterifying trehalose, which contains 8 hydroxyl groups, the total amount of esters for which the esterification rate is at least 40.0% but less than 90.0% can be calculated by totaling the area percentages (%) for the tetraester, pentaester, hexaester and heptaester calculated using the above calculation method (4), whereas the total amount of esters for which the esterification rate is less than 40.0% can be calculated by totaling the area percentages (%) for the monoester, diester and triester calculated using the above calculation method (1).

The amount of the sugar fatty acid ester composition of the component (A) within the W/O/W emulsion composition of the present invention is preferably within a range from 0.001 to 60% by mass, is more preferably from 0.01 to 40% by mass, is still more preferably from 0.1 to 20% by mass, and is most preferably from 0.1 to 10% by mass. Provided the amount of the sugar fatty acid ester composition of the component (A) within the W/O/W emulsion composition of the present invention satisfies the range described above, a highly stable W/O/W emulsion composition can be obtained for which the preparation is extremely easy and the production efficiency of the W/O/W product is favorable.

The esterification of the sugar and the fatty acid of 8 to 22 carbon atoms used for generating the sugar fatty acid ester composition of the component (A) can be performed in accordance with conventional methods, such as an esterification reaction of the sugar and the fatty acid of 8 to 22 carbon atoms, or a transesterification reaction of the sugar and an ester of the fatty acid of 8 to 22 carbon atoms.
In those cases where the esterification is performed via a transesterification with an ester of the fatty acid of 8 to 22 carbon atoms, the use of an ester of a low molecular weight alcohol such as methanol or ethanol is preferred as it facilitates the removal under reduced pressure of the alcohol generated during the transesterification reaction.
If required, an additive such as a catalyst may also be used.
There are no particular restrictions on the reaction conditions, and the esterification may be conducted with reaction conditions suitably altered in accordance with the nature of the sugar and the fatty acid of 8 to 22 carbon atoms being used, so as to achieve a hydroxyl value for the resulting sugar fatty acid ester composition that is within a range from 20 to 220.

In a specific example, when a transesterification reaction of the sugar and a methyl fatty acid is performed using dimethylsulfoxide as a solubilizing agent, the reaction is preferably conducted under reduced pressure at a reaction temperature of 70 to 120˚C, and the reaction time is preferably within a range from 8 to 12 hours. Furthermore, in the case of a microemulsion method, in which the sugar is dissolved in water, a surfactant such as a fatty acid soap or the like is used to form an emulsion with the methyl fatty acid, and the reaction is then conducted by heating under reduced pressure, the reaction is preferably conducted under reduced pressure at a reaction temperature of 90 to 170˚C, and the reaction time is preferably within a range from 24 to 60 hours. The catalyst used in the reaction is preferably an alkali catalyst such as potassium carbonate, sodium carbonate, potassium hydroxide or sodium hydroxide.

The number of hydroxyl groups varies depending on the sugar used in the reaction, and the ideal molar ratio between the sugar and the methyl fatty acid can be altered in accordance with the type of sugar used, so as to obtain a sugar fatty acid ester composition having a hydroxyl value of 20 to 220 that can be used in the present invention. For example, in the case of trehalose, the ratio of sugar : methyl fatty acid is preferably within a range from 1:3.5 to 1:7.5, in the case of lactitol and maltitol, the ratio of sugar : methyl fatty acid is preferably within a range from 1:3.7 to 1:8.5, in the case of raffinose, the ratio of sugar : methyl fatty acid is preferably within a range from 1:5.0 to 1:11.2, in the case of inositol and mannitol, the ratio of sugar : methyl fatty acid is preferably within a range from 1:2.2 to 1:5.5, in the case of xylitol, the ratio of sugar : methyl fatty acid is preferably within a range from 1:2.0 to 1:4.5, and in the case of erythritol, the ratio of sugar : methyl fatty acid is preferably within a range from 1:1.7 to 1:3.5.

As the sugar fatty acid ester composition of the component (A), products such as NOMCORT TQ-5 (manufactured by Nisshin OilliO Group, Ltd.) (trehalose isostearate esters) can be used favorably.

The W/O/W emulsion composition of the present invention includes a nonionic surfactant having an HLB value of not less than 7. Provided the W/O/W emulsion composition includes a nonionic surfactant having an HLB value of not less than 7, a highly stable W/O/W emulsion composition can be obtained for which the preparation is extremely easy and the production efficiency of the W/O/W product is favorable.

In the following description, the nonionic surfactant having an HLB value of not less than 7 is termed "component (B)".

As the component (B), a nonionic surfactant having an HLB value of not less than 7 is preferred, a nonionic surfactant having an HLB value of not less than 8 is more preferred, and a nonionic surfactant having an HLB value of 9 or greater is the most desirable.

Furthermore, as the component (B), a nonionic surfactant having an HLB value of not less than 7 and containing a polyoxyethylene group is preferred.

HLB is a value that indicates the emulsifying properties of a surfactant, and a larger value indicates a higher level of hydrophilicity, which facilitates formation of an O/W emulsion. The HLB value can usually be determined by calculation. The HLB value can be calculated using one of the following formulas, depending on the nature of the nonionic surfactant.

HLB calculation formula for an ester-based nonionic surfactant

$$HLB = 20 \times (1\text{-}S/A)$$

S: saponification value

A: neutralization number for fatty acid within ester

HLB calculation formula for an alkyl alcohol derivative nonionic surfactant (wherein the hydrophilic groups include polyhydric alcohol such as polyoxyethylene and polyglycerol)

$$HLB = (E+P)/5$$

E: mass % of polyoxyethylene within surfactant

P: mass % of polyhydric alcohol

HLB calculation formula for an alkyl alcohol derivative nonionic surfactant (wherein the hydrophilic groups are only polyoxyethylene groups)

$$HLB = E/5$$

E: mass % of polyoxyethylene within surfactant

Specific examples of the nonionic surfactant having an HLB value of not less than 7 are listed below. The nomenclature "POE (n)" is used as an abbreviation for polyoxyethylene, wherein "n" represents the polymerization degree of the polyoxyethylene.

Specific examples include polyoxyethylene sorbitan fatty acid esters such as POE (20) sorbitan monolaurate, POE (20) sorbitan monomyristate, POE (20) sorbitan monopalmitate, POE (20) sorbitan monostearate, POE (20) sorbitan monooleate, POE (20) sorbitan monoisostearate, POE (20) sorbitan tristearate, POE (20) sorbitan trioleate, POE (6) sorbitan monolaurate, POE (6) sorbitan monomyristate, POE (6) sorbitan monopalmitate, POE (6) sorbitan monostearate, POE (6) sorbitan monooleate, and POE (6) sorbitan monoisostearate; polyoxyethylene sorbitol fatty acid esters such as polyoxyethylene (30) sorbitol tetraoleate, polyoxyethylene (40) sorbitol tetraoleate, polyoxyethylene (60) sorbitol tetraoleate, polyoxyethylene (30) sorbitol tetrastearate, polyoxyethylene (40) sorbitol tetrastearate, polyoxyethylene (60) sorbitol tetrastearate, polyoxyethylene (30) sorbitol tetrabehenate, polyoxyethylene (40) sorbitol tetrabehenate, and polyoxyethylene (60) sorbitol tetrabehenate; polyoxyethylene glycerol fatty acid esters such as POE (5) glyceryl monostearate, POE (15) glyceryl monostearate, POE (5) glyceryl monooleate, POE (15) glyceryl monooleate, POE (5) glyceryl monoisostearate, POE (15) glyceryl monoisostearate, POE (10) glyceryl tristearate, POE (20) glyceryl tristearate, POE (30) glyceryl tristearate, POE (10) glyceryl trioleate, POE (20) glyceryl trioleate, POE (30) glyceryl trioleate, POE (10) glyceryl triisostearate, POE (20) glyceryl triisostearate, and POE (30) glyceryl triisostearate; polyglycerol fatty acid esters such as triglyceryl monolaurate, tetraglyceryl monolaurate, pentaglyceryl monolaurate, decaglyceryl monolaurate, triglyceryl monomyristate, tetraglyceryl monomyristate, pentaglyceryl monomyristate, decaglyceryl monomyristate, triglyceryl monopalmitate, tetraglyceryl monopalmitate, pentaglyceryl monopalmitate, decaglyceryl monopalmitate, triglyceryl monostearate, tetraglyceryl monostearate, pentaglyceryl monostearate, decaglyceryl monostearate, triglyceryl monooleate, tetraglyceryl monooleate, pentaglyceryl monooleate, decaglyceryl monooleate, triglyceryl monoisostearate, tetraglyceryl monoisostearate, pentaglyceryl monoisostearate, decaglyceryl monoisostearate, decaglyceryl dilaurate, decaglyceryl trilaurate, decaglyceryl tetralaurate, decaglyceryl dimyristate, decaglyceryl trimyristate, decaglyceryl tetramyristate, decaglyceryl dipalmitate, decaglyceryl tripalmitate, decaglyceryl tetrapalmitate, decaglyceryl distearate, decaglyceryl tristearate, decaglyceryl tetrastearate, decaglyceryl dioleate, decaglyceryl trioleate, decaglyceryl tetraoleate, decaglyceryl diisostearate, decaglyceryl triisostearate, and decaglyceryl tetraisostearate; polyoxyethylene alkyl ethers such as polyoxyethylene hydrogenated castor oil in which the number of added mols of ethylene oxide is 15 or greater, polyoxyethylene castor oil in which the number of added mols of ethylene oxide is 15 or greater, polyoxyethylene lauryl ether in which the number of added mols of ethylene oxide is 3 or greater, polyoxyethylene cetyl ether in which the number of added mols of ethylene oxide is 3 or greater, polyoxyethylene stearyl ether in which the number of added mols of ethylene oxide is 4 or greater, polyoxyethylene oleyl ether in which the number of added mols of ethylene oxide is 4 or greater, polyoxyethylene isostearyl ether in which the number of added mols of ethylene oxide is 4 or greater, and polyoxyethylene behenyl ether in which the number of added mols of ethylene oxide is 4 or greater; polyethylene glycol fatty acid esters such as polyethylene glycol monolaurate in which the number of added mols of ethylene oxide is 3 or greater, polyethylene glycol monostearate in which the number of added mols of ethylene oxide is 4 or greater, polyethylene glycol monooleate in which the number of added mols of ethylene oxide is 4 or greater, polyethylene glycol monoisostearate in which the number of added mols of ethylene oxide is 4 or greater, and polyethylene glycol monobehenate in which the number of added mols of ethylene oxide is 5 or greater; and polyoxyethylene-polyoxypropylene alkyl ethers such as polyoxyethylene (3) polyoxypropylene (2) decyl ether, polyoxyethylene (12) polyoxypropylene (2) decyl ether, and polyoxyethylene (20) polyoxypropylene (2) cetyl ether. Of these, polyoxyethylene sorbitan fatty acid esters, polyoxyethylene hydrogenated castor oil in which the number of added mols of ethylene oxide is 15 or greater, polyoxyethylene cetyl ether in which the number of added mols of ethylene oxide is 3 or greater, polyoxyethylene stearyl ether in which the number of added mols of ethylene oxide is 4 or greater, polyoxyethylene oleyl ether in which the number of added mols of ethylene oxide is 4 or greater, polyoxyethylene isostearyl ether in which the number of added mols of ethylene oxide is 4 or greater, polyoxyethylene behenyl ether in which the number of added mols of ethylene oxide is 4 or greater, polyethylene glycol monostearate in which the number of added mols of ethylene oxide is 4 or greater, polyethylene glycol monooleate in which the number of added mols of ethylene oxide is 4 or greater, polyethylene glycol monoisostearate in which the number of added mols of ethylene oxide is 4 or greater, and polyethylene glycol monobehenate in which the number of added mols of ethylene oxide is 5 or greater are preferred. These nonionic surfactants having an HLB value of not less than 7 may be used individually, or in combinations containing two or more different surfactants. By using one of the above surfactants as the nonionic surfactant having an HLB value of not less than 7 of the component (B), a highly stable W/O/W emulsion composition can be obtained for which the preparation is extremely easy and the production efficiency of the W/O/W product is favorable.

By using a polyoxyethylene hydrogenated castor oil in which the number of added mols of ethylene oxide is 15 or greater as the nonionic surfactant having an HLB value of not less than 7 of the component (B), a W/O/W emulsion composition with superior water retention properties can be obtained.

The amount of the nonionic surfactant having an HLB value of not less than 7 of the component (B) within the W/O/W emulsion composition of the present invention is preferably within a range from 0.001 to 10% by mass, more preferably from 0.01 to 8% by mass, still more preferably from 0.05 to 4% by mass, and most preferably from 0.1 to 2.5% by mass. Provided the amount of the nonionic surfactant having an HLB value of not less than 7 of the component (B) within the W/O/W emulsion composition of the present invention satisfies the above range, a highly stable W/O/W emulsion composition can be obtained for which the preparation is extremely easy and the production efficiency of the W/O/W product is favorable.

The mass ratio between the sugar fatty acid ester composition of the component (A) and the nonionic surfactant having an HLB value of not less than 7 of the component (B) within the W/O/W emulsion composition of the present invention, reported as a mass ratio of (sugar fatty acid ester composition of the component (A)) : (nonionic surfactant having an HLB value of not less than 7 of the component (B)), is preferably within a range from 1:0.01 to 1:1.4, more preferably from 1:0.02 to 1:1.2, and most preferably from 1:0.05 to 1:1.0. Provided the mass ratio between the sugar fatty acid ester composition of the component (A) and the nonionic surfactant having an HLB value of not less than 7 of the component (B) within the W/O/W emulsion composition of the present invention satisfies the above range, a highly stable W/O/W emulsion composition can be obtained for which the preparation is extremely easy and the production efficiency of the W/O/W product is favorable.

A polyhydric alcohol may be blended into the W/O/W emulsion composition of the present invention. By blending a polyhydric alcohol into the W/O/W emulsion composition of the present invention, the dispersibility of the inner water phase can be improved, thereby improving the production efficiency and the stability of the W/O/W product. Hereafter the polyhydric alcohol is termed "component (C)".

Specific examples of the polyhydric alcohol of the component (C) include glycerol, diglycerol, triglycerol, polyethylene glycol, propylene glycol, 1,3-butylene glycol, 3-methyl-1,3-butanediol, 1,2-pentanediol, 1,2-hexanediol, hexylene glycol, sorbitol, erythritol and xylitol. Glycerol, propylene glycol, 1,3-butylene glycol and 3-methyl-1,3-butanediol are particularly preferred. These polyhydric alcohols may be used individually or in combinations containing two or more different alcohols. By using one or more of the above compounds as the polyhydric alcohol, the production efficiency of the W/O/W product can be further enhanced, and the stability can be further improved.

The amount of the polyhydric alcohol of the component (C) within the W/O/W emulsion composition of the present invention is preferably within a range from 0.1 to 45% by mass, more preferably from 0.5 to 40% by mass, and most preferably from 1 to 35% by mass. Provided the amount of the polyhydric alcohol of the component (C) within the W/O/W emulsion composition of the present invention satisfies the above range, improvements can be expected in the production efficiency and stability of the W/O/W product.

The W/O/W emulsion composition of the present invention contains water. Examples of the water used in the W/O/W emulsion composition of the present invention include purified water such as distilled water and ion-exchanged water, water obtained from fruit or flowers, and water obtained by purifying seawater or the like. These waters may be used individually or in combinations containing two or more different types of water. Hereafter the water is termed "component (D)".

Although there are no particular restrictions on the amount of water within the W/O/W emulsion composition of the present invention, the amount is preferably within a range from 30 to 99% by mass, more preferably from 35 to 90% by mass, and most preferably from 40 to 85% by mass.

Various other components used in conventional W/O/W emulsion compositions may be added as required to the W/O/W emulsion composition of the present invention, for purposes such as improving the functionality, imparting superior moisture retention, providing skin nutrients or preventing quality degradation, provided the addition of these other components does not impair the effects of the present invention. Specific examples of these other components include other surfactants such as nonionic surfactants having an HLB value of less than 7, anionic surfactants, cationic surfactants and amphoteric surfactants, oily components such as phospholipids, hydrocarbon oils, ester oils, waxes and silicones, powdered components such as inorganic pigments, organic pigments, iron oxide and talc, as well as moisturizers, natural water-soluble polymers, semi-synthetic water-soluble polymers, synthetic water-soluble polymers, inorganic water-soluble polymers, ultraviolet absorbers, sequestering agents, lower alcohols, monosaccharides, oligosaccharides, polysaccharides, amino acids, organic amines, synthetic resin emulsions, salts, pH regulators, vitamins, plant extracts, antioxidants, antioxidant assistants and fragrances. These components may be used individually or in combinations of two or more different components.

The W/O/W emulsion composition of the present invention can be produced using a typical phase inversion emulsification. In a specific example, an oil phase is prepared by heating and dissolving, at a temperature of 60 to 90˚C, a mixture of the sugar fatty acid ester composition of the component (A), the nonionic surfactant having an HLB value of not less than 7 of the component (B), any other optional surfactants that may be added, and any other oily components. Further, a water phase is prepared by heating and dissolving, at a temperature of 60 to 90˚C, the water, the polyhydric alcohol, and any other water-based components. The water phase is adjusted to a temperature of 60 to 90˚C and then added gradually to the oil phase with constant stirring using a disper, homomixer or propeller blade or the like, and following completion of the addition of the water phase, the mixture is cooled to room temperature, and any water-based components that are susceptible to degradation under higher temperatures are added following cooling, thus completing the production of the W/O/W emulsion composition. A W/O emulsion is generated immediately following commencement of the addition of the water phase, and then during the addition of the water phase or during cooling, when the continuous layer changes from the oil phase to the water phase, the phase change occurs with a water phase still encapsulated within the oil phase, meaning a W/O/W emulsion composition is formed via a single-stage emulsification method.

The blend ratio between the oil phase and the water phase preferably yields a mass ratio of oil phase : water phase that is within a range from 1:99 to 70:30, and this ratio of oil phase : water phase is more preferably from 1:99 to 60:40, and is most preferably from 1:99 to 50:50.

The W/O/W emulsion composition of the present invention contains a plurality of water phases dispersed within oil droplets, and exhibits excellent production efficiency of the W/O/W product. In the W/O/W emulsion composition of the present invention, the proportion of oil droplets that contain a water phase therein is preferably not less than 50%, and this proportion of oil droplets that contain a water phase therein is more preferably 80% or greater.

Furthermore, when the W/O/W emulsion composition of the present invention is stored for one week at a temperature of 40˚C, the state of dispersion of the inner water phase undergoes no substantial change, indicating that the emulsion composition exhibits excellent emulsion stability over long periods.

Moreover, because the W/O/W emulsion composition of the present invention can be obtained by a phase inversion emulsification method that has recently become widespread, preparation of the emulsion composition is extremely simple. Because production of the W/O/W emulsion composition of the present invention does not require a two-stage emulsification, a number of production steps can be eliminated, and new production facilities such as emulsification equipment is unnecessary, meaning the invention is also advantageous from the perspective of production costs.

The W/O/W emulsion composition of the present invention is ideal for external application to the skin, and can be used without any modification in cosmetic preparations, quasi-drugs and pharmaceutical products and the like. The W/O/W emulsion composition of the present invention is particularly useful in cosmetic preparations.

Further, the W/O/W emulsion composition of the present invention may also be used as a premixed composite raw material, and may be incorporated within cosmetic preparations, quasi-drugs and pharmaceutical products and the like as one composite raw material. The W/O/W emulsion composition of the present invention is particularly ideal for addition to cosmetic preparations.

In those cases where the W/O/W emulsion composition of the present invention is used, as is, as a cosmetic preparation, and those cases where the W/O/W emulsion composition of the present invention is added to a cosmetic preparation, although there are no particular restrictions on the form of the cosmetic preparation, milky lotions, cream, essence, lotion, ointment or pack or the like is ideal.

Various other components typically used in conventional cosmetic preparations may be added as required to a cosmetic preparation containing the W/O/W emulsion composition of the present invention, for purposes such as improving the functionality, imparting superior moisture retention, providing skin nutrients or preventing quality degradation, provided the addition of these other components does not impair the effects of the present invention. Specific examples of these other components include surfactants such as nonionic surfactants having an HLB value of less than 7, anionic surfactants, cationic surfactants and amphoteric surfactants, oily components such as phospholipids, hydrocarbon oils, ester oils, waxes and silicones, powdered components such as inorganic pigments, organic pigments, iron oxide and talc, as well as moisturizers, natural water-soluble polymers, semi-synthetic water-soluble polymers, synthetic water-soluble polymers, inorganic water-soluble polymers, ultraviolet absorbers, sequestering agents, lower alcohols, monosaccharides, oligosaccharides, polysaccharides, amino acids, organic amines, synthetic resin emulsions, salts, pH regulators, vitamins, plant extracts, antioxidants, antioxidant assistants and fragrances. These components may be used individually or in combinations of two or more different components.

A cosmetic preparation containing the W/O/W emulsion composition of the present invention can be produced using conventional cosmetic preparation production methods.

Examples

A more detailed description of the present invention is presented below based on a series of examples and comparative examples, although the present invention is in no way limited by these examples.

Production of a trehalose isostearate composition 2 (T-iSt2)

A 2,000 ml four-neck flask fitted with a stirrer, a thermometer, a nitrogen gas inlet equipped with a stopcock, and a glass tube equipped with a stopcock was charged with 241.9 g (0.64 mol) of trehalose dihydrate (TOREHA HT, manufactured by Hayashibara Group), 1,001.9 g (3.33 mol) of methyl isostearate (prepared by normal methods, acid value: 2.0), 3.0 g of potassium carbonate (potassium carbonate, manufactured by Wako Pure Chemical Industries, Ltd.), and 62.2 g of sodium stearate (sodium stearate, manufactured by Wako Pure Chemical Industries, Ltd.), and the mixture was stirred at 95˚C while moisture was removed by drying under reduced pressure. The inside of the four-neck flask was returned to normal pressure using nitrogen gas, 12.1 g of potassium carbonate (potassium carbonate, manufactured by Wako Pure Chemical Industries, Ltd.) was added as a catalyst, the flask was once again evacuated to a state of reduced pressure, and the resulting mixture was reacted by stirring under reduced pressure for 48 hours at a temperature of 110 to 170˚C. Following completion of the reaction, the reaction mixture was diluted with 1,000 ml of xylene and 500 ml of butyl acetate, and then filtered. The filtrate was washed repeatedly and carefully with hot water until the lower water solution layer was substantially neutral. Following completion of this washing, the upper xylene layer was dried under reduced pressure, the xylene was removed by distillation, and the residue was subjected to a decolorization treatment using activated carbon and activated clay. The product was then subjected to deodorizing and distillation treatments using normal methods, thus yielding 705 g of the target trehalose isostearate composition 2 having a hydroxyl value of 94.

Other sugar fatty acid ester compositions were produced using the same method, using the sugar and fatty acid varieties listed in Table 4 and Table 5 with various selected molar ratios between the sugar and the fatty acid. The sugar fatty acid ester compositions labeled No. 1 to No. 21 in Table 4 represent the compositions used in examples of the present invention. Further, the sugar fatty acid ester compositions labeled No. 22 to No. 28 in Table 5 represent the compositions used in comparative examples.

The sugars and fatty acids listed in Table 4 and Table 5 used the products listed below.

Trehalose: TOREHA HT, manufactured by Hayashibara Group Lactitol: LACTITOL NIKKEN, manufactured by Nikken Fine Chemical Co., Ltd.

Raffinose: D(+)-raffinose pentahydrate, manufactured by Wako Pure Chemical Industries, Ltd.

Inositol: myo-Inositol, manufactured by Wako Pure Chemical Industries, Ltd.

Maltitol: MALBIT, manufactured by Nikken Fine Chemical Co., Ltd.

Mannitol: MANNITOL NIKKEN, manufactured by Nikken Fine Chemical Co., Ltd.

Xylitol: XYLITOL NIKKEN, manufactured by Nikken Fine Chemical Co., Ltd.

Erythritol: erythritol, manufactured by Nikken Fine Chemical Co., Ltd.

Isostearic acid: EMASOL 874, manufactured by Cognis GmbH

Lauric acid: NAA-122, manufactured by NOF Corporation

Palmitic acid: NAA-160, manufactured by NOF Corporation

Oleic acid: NAA-34, manufactured by NOF Corporation

2-hexyldecanoic acid: ISOCARB 16, manufactured by Sasol Ltd.

2-decyltetradecanoic acid: ISOCARB 24, manufactured by Sasol Ltd.

Stearic acid: NAA-172, manufactured by NOF Corporation

The sugar fatty acid esters were abbreviated using the following abbreviations.

Trehalose isostearate composition: T-iSt

Trehalose laurate composition: T-La

Trehalose palmitate composition: T-Pa

Trehalose oleate composition: T-Ol

Trehalose 2-hexyldecanoate composition: T-HD

Lactitol isostearate composition: LT-iSt

Lactitol 2-decyltetradecanoate composition: LT-DT

Raffinose isostearate composition: RA-iSt

Raffinose stearate composition: RA-St

Raffinose 2-ethylhexanoate composition: RA-EH

Inositol isostearate composition: IN-iSt

Maltitol isostearate composition: MT-iSt

Mannitol isostearate composition: MN-iSt

Xylitol isostearate composition: X-iSt

Erythritol isostearate composition: E-iSt

[Table 4]

| Table 4: Hydroxyl values, raw materials, and reaction molar ratios for sugar fatty acid ester compositions | | | | | |
|---|---|---|---|---|---|
| No | Sugar fatty acid ester composition | Hydroxyl value | Sugar | Fatty cid | Reaction molar ratio (sugar: fatty acid) |
| 1 | T-iSt1 | 142 | Trehalose | Isostearic acid | 1:3.9 |
| 2 | T-iSt2 | 94 | Trehalose | Isostearic acid | 1:5.2 |
| 3 | T-iSt3 | 57 | Trehalose | Isostearic acid | 1:6.8 |
| 4 | T-iSt4 | 42 | Trehalose | Isostearic acid | 1:7.3 |
| 5 | T-La | 36 | Trehalose | Lauric acid | 1:7.3 |
| 6 | T-Pa | 110 | Trehalose | Palmitic acid | 1:5.2 |
| 7 | T-Ol | 95 | Trehalose | Oleic acid | 1:5.2 |
| 8 | T-HD | 100 | Trehalose | 2-hexyldecanoic acid | 1:5.2 |
| 9 | LT-iSt1 | 189 | Lactitol | Isostearic acid | 1:3.9 |

(continued)

| No | Sugar fatty acid ester composition | Hydroxyl value | Sugar | Fatty cid | Reaction molar ratio (sugar: fatty acid) |
|---|---|---|---|---|---|
| 10 | LT-iSt2 | 57 | Lactitol | Isostearic acid | 1:7.3 |
| 11 | LT-DT | 59 | Lactitol | 2-decyltetradecanoic acid | 1:6.8 |
| 12 | RA-iSt1 | 124 | Raffinose | Isostearic acid | 1:6.8 |
| 13 | RA-iSt2 | 53 | Raffinose | Isostearic acid | 1:8.2 |
| 14 | RA-iSt3 | 21 | Raffinose | Isostearic acid | 1:11.0 |
| 15 | RA-St | 130 | Raffinose | Stearic acid | 1:6.8 |
| 16 | RA-EH | 101 | Raffinose | 2-ethylhexanoic acid | 1:8.9 |
| 17 | IN-iSt | 80 | Inositol | Isostearic acid | 1:5.0 |
| 18 | MT-iSt | 57 | Maltitol | Isostearic acid | 1:7.0 |
| 19 | MN-iSt | 27 | Mannitol | Isostearic acid | 1:5.0 |
| 20 | X-iSt | 36 | Xylitol | Isostearic acid | 1:4.0 |
| 21 | E-iSt | 51 | Erythritol | Isostearic acid | 1:3.0 |

Table 4: Hydroxyl values, raw materials, and reaction molar ratios for sugar fatty acid ester compositions

[Table 5]

| No | Sugar fatty acid ester composition | Hydroxyl value | Sugar | Fatty acid | Reaction molar ratio (sugar : fatty acid) |
|---|---|---|---|---|---|
| 22 | T-iSt5 | 350 | Trehalose | Isostearic acid | 1:1.7 |
| 23 | T-iSt6 | 238 | Trehalose | Isostearic acid | 1:3.0 |
| 24 | T-iSt7 | 5 | Trehalose | Isostearic acid | 1:8.5 |
| 25 | LT-iSt3 | 301 | Lactitol | Isostearic acid | 1:3.5 |
| 26 | LT-iSt4 | 6 | Lactitol | Isostearic acid | 1:9.5 |
| 27 | RA-iSt4 | 335 | Raffinose | Isostearic acid | 1:3.0 |
| 28 | RA-iSt5 | 5 | Raffinose | Isostearic acid | 1:11.5 |

Table 5: Hydroxyl values, raw materials, and reaction molar ratios for sugar fatty acid ester compositions

Examples of the amounts of the esters of each esterification rate within the above sugar fatty acid ester compositions are illustrated in Table 6 (trehalose isostearate compositions), Table 7 (lactitol isostearate compositions) and Table 8 (raffinose isostearate composition).
The amount of the ester of each esterification rate within the sugar fatty acid ester composition is recorded as an area percentage (%) for that ester calculated by HPLC analysis using a combination of the GPC columns and ODS column described below.

[HPLC Measurement Conditions]

The HPLC that used GPC columns to calculate area % values for the monoester, diester, triester and polyesters within the sugar fatty acid ester composition was conducted under the conditions described below.
Four GPC columns were connected in the sequence listed below: TSK-GEL G4000HHR, 5 μm, 7.8 × 300 mm, TSK-GEL G3000HHR, 5 μm, 7.8 × 300 mm, TSK-GEL G2000HHR, 5 μm, 7.8 × 300 mm, and TSK-GEL G3000HHR, 5 μm, 7.8 × 300 mm. Furthermore, the HPLC analysis was performed using a Shimadzu high-performance liquid chromatograph feed unit LC-10AD manufactured by Shimadzu Corporation, a Shimadzu high-performance liquid chromatograph column oven CTO-10A, and a Shimadzu high-performance liquid chromatograph differential refractive index detector.

HPLC measurement conditions using GPC columns

Columns: four series-connected GPC columns of internal diameter 7.8 mm and length 300 mm packed with a 5 $\mu$m styrenedivinylbenzene substrate

Mobile phase: tetrahydrofuran

Column temperature: 40˚C

Mobile phase flow rate: 0.5 mL/min

Detection: differential refractive index (RI)

The HPLC that used an ODS column to calculate the proportion of each of the esters within the polyester fraction of the sugar fatty acid ester composition of the component (A) was conducted under the conditions described below.

The ODS column used was a Kaseisorb LC ODS2000, 5 $\mu$m, 4.6 × 150 mm, manufactured by Tokyo Chemical Industry Co., Ltd. The HPLC analysis was performed using a Shimadzu high-performance liquid chromatograph feed unit LC-10AD manufactured by Shimadzu Corporation, a Shimadzu high-performance liquid chromatograph column oven CTO-10A, and a Shimadzu high-performance liquid chromatograph differential refractive index detector.

HPLC measurement conditions using ODS column

Column: an ODS column of internal diameter 4.6 mm and length 150 mm packed with a 5 $\mu$m substrate

Mobile phase: tetrahydrofuran : methanol = 55:45 (volume ratio)

Column temperature: 40˚C

Mobile phase flow rate: 0.8 mL/min

Detection: differential refractive index (RI)

The area percentages (%) of each of the esters within the sugar fatty acid ester composition were calculated on the basis of the HPLC measurement results, using the calculation methods described above.

[Table 6]

Table 6: Amounts of esters of each esterification rate within trehalose isostearate compositions

| Sugarfatty acid ester composition | Hydroxyl value | Esterification rate | | | | | | | | |
|---|---|---|---|---|---|---|---|---|---|---|
| | | 0.0% raw material | 12.5% *1 | 25.0% *2 | 37.5% *3 | 50.0% *4 | 62.5% *5 | 75.0% *6 | 87.5% *7 | 100% *8 |
| T-iSt1 | 142 | 0.5% | 0.3% | 6.0% | 17.1% | 27.5% | 25.7% | 15.0% | 6.5% | 1.4% |
| | | 23.9% | | | | 74.7% | | | | 1.4% |
| T-iSt2 | 94 | 0.0% | 0.0% | 1.2% | 7.5% | 18.6% | 25.3% | 23.9% | 16.1% | 7.4% |
| | | 8.7% | | | | 83.9% | | | | 7.4% |
| T-iSt3 | 57 | 0.0% | 0.0% | 0.0% | 3.5% | 8.9% | 20.8% | 26.2% | 26.6% | 14.0% |
| | | 3.5% | | | | 82.5% | | | | 14.0% |
| T-iSt4 | 42 | 0.0% | 0.0% | 0.0% | 0.7% | 3.8% | 13.1% | 23.3% | 33.0% | 26.1% |
| | | 0.7% | | | | 73.2% | | | | 26.1% |
| T-iSt5 | 350 | 0.0% | 20.7% | 34.1% | 26.8% | 12.4% | 5.1% | 0.9% | 0.0% | 0.0% |
| | | 81.6% | | | | 18.4% | | | | 0.0% |
| T-iSt6 | 238 | 4.8% | 5.0% | 21.4% | 29.3% | 22.5% | 12.0% | 3.8% | 0.9% | 0.3% |
| | | 60.5% | | | | 39.2% | | | | 0.3% |
| T-iSt7 | 5 | 0.0% | 0.0% | 0.0% | 0.0% | 0.0% | 1.0% | 2.9% | 17.4% | 78.7% |
| | | 0.0% | | | | 21.3% | | | | 78.7% |

*1 monoester, *2 diester, *3 triester, *4 tetraester, *5 pentaester, *6 hexaester, *7 heptaester, *8 octaester

EP 2 172 263 A1

[Table 7]

Table 7: Amounts of esters of each esterification rate within lactitol isostearate compositions

| Sugar fatty acid ester composition | Hydroxyl value | Esterification rate | | | | | | | | | |
|---|---|---|---|---|---|---|---|---|---|---|---|
| | | 0.0% raw material | 11.1% *1 | 22.2% *2 | 33.3% *3 | 44.4% *4 | 55.6% *5 | 66.7% *6 | 77.8% *7 | 88.9% *8 | 100% *9 |
| LT-iSt2 | 57 | 0.0% | 0.0% | 0.0% | 0.0% | 2.7% | 10.0% | 17.6% | 25.3% | 30.1% | 14.3% |
| | | 0.0% | | | | 85.7% | | | | | 14.3% |
| LT-iSt3 | 301 | 3.3% | 8.8% | 25.3% | 31.6% | 20.6% | 8.0% | 2.4% | 0.0% | 0.0% | 0.0% |
| | | 69.0% | | | | 31.0% | | | | | 0.0% |

*1 monoester, *2 diester, *3 triester, *4 tetraester, *5 pentaester, *6 hexaester, *7 heptaester, *8 octaester, *9 nonaester

EP 2 172 263 A1

[Table 8]

| Table 8: Amounts of esters of each esterification rate within raffinose isostearate composition | | | | | | | | | | | | | |
|---|---|---|---|---|---|---|---|---|---|---|---|---|---|
| Sugar fatty acid ester composition | Hydroxyl value | Esterification rate | | | | | | | | | | | |
| | | 0.0% raw material | 9.1% *1 | 18.2% *2 | 27.3% *3 | 36.4% *4 | 45.5% *5 | 54.5% *6 | 63.6% *7 | 72.7% *8 | 81.8% *9 | 90.9% *10 | 100% *11 |
| RA-iSt2 | 53 | 1.6% | 0.0% | 0.7% | 1.6% | 2.1% | 8.3% | 10.7% | 16.0% | 19.6% | 22.0% | 14.4% | 3.0% |
| | | 6.0% | | | | | 76.6% | | | | | 17.4% | |
| *1 monoester, *2 diester, *3 triester, *4 tetraester, *5 pentaester, *6 hexaester, *7 heptaester, *8 octaester, *9 nonaester, *10 decaester, *11 undecaester | | | | | | | | | | | | | |

[Evaluation of W/O/W emulsion compositions]

W/O/W emulsion compositions were prepared using the formulations shown below in Tables 9 to 16, and the emulsion state of each of the thus obtained W/O/W emulsion compositions was observed using a microscope. The W/O/W emulsion compositions were then stored at 40˚C for one month, and the emulsion state following storage was once again observed under a microscope.

[Method of preparing W/O/W emulsion compositions]

The components that constitute the oil phase were combined in a beaker, and the resulting mixture was then heated and dissolved at 70˚C to prepare the oil phase. The components that constitute the water phase were combined in a separate beaker, and the resulting mixture was then heated and dissolved at 70˚C to prepare the water phase. Then, with the oil phase undergoing constant stirring at 500 rpm using a desktop disper, the water phase was added gradually to the oil phase, and once the continuous layer was confirmed as having changed to the water phase, the additives were added to complete the preparation of the W/O/W emulsion composition.

[Method and criteria for evaluating the production efficiency of the W/O/W emulsion composition]

Immediately following preparation, each of the W/O/W emulsion compositions was dripped onto a slide glass, and a cover glass was then placed on top to generate a thin film and complete preparation of an evaluation sample. The emulsion state of the evaluation sample was observed at a magnification of 1,000× using a microscope (DIGITAL MICROSCOPE VHX-500, manufactured by Keyence Corporation), and the proportion of oil droplets within the field of view that contained an inner water phase was determined visually.
◎: 80% or more of the oil droplets contained an inner water phase.
○: not less than 50% but less than 80% of the oil droplets contained an inner water phase.
□: not less than 30% but less than 50% of the oil droplets contained an inner water phase.
△: less than 30% of the oil droplets contained an inner water phase.
×: absolutely no inner water phase was observed.

[Method and criteria for evaluating the stability of the W/O/W emulsion composition]

Following storage at 40˚C for one month, each of the prepared W/O/W emulsion compositions was dripped onto a slide, and a cover glass was then placed on top to generate a thin film and complete preparation of an evaluation sample. The emulsion state of the evaluation sample was observed at a magnification of 1,000× using a microscope (DIGITAL MICROSCOPE VHX-500, manufactured by Keyence Corporation), the observed state was compared with that prior to the storage period, and the degree of change in the emulsion state was evaluated.
◎: no substantial change from prior to storage.
○: slight combination of some inner water phases was observed, but no substantial change in the proportion of oil droplets containing an inner water phase.
□: slight reduction in the proportion of oil droplets containing an inner water phase.
△: reduction in the proportion of oil droplets containing an inner water phase to less than one half of that prior to storage.
×: all inner water phases had disappeared. Alternatively, no water phase existed even prior to storage.
[Table 9]

| Table 9: W/O/W emulsion composition formulations (% by mass) and evaluation results | | Example 1 | Example 2 | Example 3 | Example 4 | Comparative example 1 | Comparative example 2 |
|---|---|---|---|---|---|---|---|
| | Component | | | | | | |
| Oil phase | IN-iSt (hydroxyl value: 80) | 5 | - | - | - | - | 5 |
| | T-iSt2 (hydroxyl value: 94) | - | 5 | 10 | - | - | - |
| | LT-iSt2 (hydroxyl value: 57) | - | - | - | 5 | - | - |
| | Polyoxyethylene (20) sorbitan monooleate *1 | 0.7 | 0.7 | 0.7 | 0.7 | 0.7 | - |
| | Sorbitan sesquioleate *2 | 0.3 | 0.3 | 0.3 | 0.3 | 0.3 | 0.3 |
| | Cetyl 2-ethylhexanoate | 5 | 5 | - | 5 | 10 | 5 |
| Water phase | Pure water | 79.6 | 79.6 | 79.6 | 79.6 | 79.6 | 80.3 |
| | methylparaben | 0.1 | 0.1 | 0.1 | 0.1 | 0.1 | 0.1 |
| Additive | 1% aqueous solution of sodium hydroxide | 2.6 | 2.6 | 2.6 | 2.6 | 2.6 | 2.6 |
| | 1.5% aqueous solution of carbomer *3 | 6.7 | 6.7 | 6.7 | 6.7 | 6.7 | 6.7 |
| Evaluation | W/O/W production efficiency | ◎ | ◎ | ◎ | ◎ | × | × |
| | Stability | ○ | ○ | ○ | ○ | × | × |

*1  RHEODOL TW-O120, manufactured by Kao Corporation, HLB: 15.0

*2  RHEODOL AO-15, manufactured by Kao Corporation, HLB: 3.7

*3  CARBOPOL 940, manufactured by Noveon Corporation

[Table 10]

Table 10: W/O/W emulsion composition formulations (% by mass) and evaluation results

| | Component | Example 5 | Example 6 | Example 7 | Example 8 |
|---|---|---|---|---|---|
| Oil phase | RA-iSt2 (hydroxyl value: 53) | 5 | - | - | - |
| | MN-iSt (hydroxyl value: 27) | - | 5 | - | - |
| | X-iSt (hydroxyl value: 36) | - | - | 5 | - |
| | E-iSt (hydroxyl value: 51) | - | - | - | 5 |
| | Polyoxyethylene (20) sorbitan monooleate *1 | 0.7 | 0.7 | 0.7 | 0.7 |
| | Sorbitan sesquioleate *2 | 0.3 | 0.3 | 0.3 | 0.3 |
| | Cetyl 2-ethylhexanoate | 5 | 5 | 5 | 5 |
| Water phase | Pure water | 79.6 | 79.6 | 79.6 | 79.6 |
| | methylparaben | 0.1 | 0.1 | 0.1 | 0.1 |
| Additive | 1% aqueous solution of sodium hydroxide | 2.6 | 2.6 | 2.6 | 2.6 |
| | 1.5% aqueous solution of carbomer *3 | 6.7 | 6.7 | 6.7 | 6.7 |
| Evaluation | W/O/W production efficiency | ◎ | ○ | ○ | ○ |
| | Stability | ○ | ○ | ○ | ○ |

*1 RHEODOL TW-O120, manufactured by Kao Corporation, HLB: 15.0

*2 RHEODOL AO-15, manufactured by Kao Corporation, HLB: 3.7

*3 CARBOPOL 940, manufactured by Noveon Corporation

As is evident from Tables 9 and 10, the W/O/W emulsion compositions of examples 1 to 8 were prepared using a widely used conventional phase inversion emulsification sequence, and yet exhibited a high production efficiency for the W/O/W emulsion product and favorable composition stability. In contrast, in the comparative examples 1 and 2, which lacked either the sugar fatty acid ester composition or the nonionic surfactant having an HLB value of not less than 7 that represent the essential components of the present invention, absolutely no W/O/W emulsion product was generated.
[Table 11]

| Table 11: W/O/W emulsion composition formulations (% by mass) and evaluation results | | Example 9 | Example 10 | Example 11 | Example 12 | Example 13 | Comparative example 3 | Comparative example 4 | Comparative example 5 |
|---|---|---|---|---|---|---|---|---|---|
| | Component | | | | | | | | |
| Oil phase | T-iSt2 (hydroxyl value: 94) | 5 | 5 | 5 | 5 | 5 | - | - | - |
| | Polyoxyethylene (20) sorbitan monooleate *1 | 0.7 | 0.7 | 0.7 | 0.7 | 0.7 | 0.7 | 0.7 | 0.7 |
| | Sorbitan sesquioleate *2 | 0.3 | 0.3 | 0.3 | 0.3 | 0.3 | 0.3 | 0.3 | 0.3 |
| | Cetyl 2-ethylhexanoate | 5 | 5 | 5 | 5 | 5 | 5 | 10 | 10 |
| Water phase | Pure water | 74.6 | 69.6 | 69.6 | 59.6 | 49.6 | 79.6 | 69.6 | 49.6 |
| | Glycerol | 5 | 10 | - | - | 10 | 5 | - | 10 |
| | 1,3-butylene glycol | - | - | 10 | 20 | 20 | - | 10 | 20 |
| | methylparaben | 0.1 | 0.1 | 0.1 | 0.1 | 0.1 | 0.1 | 0.1 | 0.1 |
| Additive | 1% aqueous solution of sodium hydroxide | 2.6 | 2.6 | 2.6 | 2.6 | 2.6 | 2.6 | 2.6 | 2.6 |
| | 1.5% aqueous solution of carbomer *3 | 6.7 | 6.7 | 6.7 | 6.7 | 6.7 | 6.7 | 6.7 | 6.7 |
| Evaluation | W/O/W production efficiency | ◎ | ◎ | ◎ | ◎ | ◎ | × | × | × |
| | Stability | ◎ | ◎ | ◎ | ◎ | ◎ | × | × | × |

*1  RHEODOL TW-O120, manufactured by Kao Corporation, HLB: 15.0

*2  RHEODOL AO-15, manufactured by Kao Corporation, HLB: 3.7

*3  CARBOPOL 940, manufactured by Noveon Corporation

[Table 12]

| Table 12: W/O/W emulsion composition formulations (% by mass) and evaluation results | | | | | | |
|---|---|---|---|---|---|
| | Component | Example 14 | Example 15 | Example 16 | Example 17 |
| Oil phase | IN-iSt (hydroxyl value: 80) | 5 | - | - | - |
| | LT-iSt2 (hydroxyl value: 57) | - | 5 | - | - |
| | RA-iSt2 (hydroxyl value: 53) | - | - | 5 | - |
| | MN-iSt (hydroxyl value: 27) | - | - | - | 5 |
| | Polyoxyethylene (20) sorbitan monooleate *1 | 0.7 | 0.7 | 0.7 | 0.7 |
| | Sorbitan sesquioleate *2 | 0.3 | 0.3 | 0.3 | 0.3 |
| | Cetyl 2-ethylhexanoate | 5 | 5 | 5 | 10 |
| Water phase | Pure water | 49.6 | 49.6 | 49.6 | 44.6 |
| | Glycerol | 10 | 10 | 10 | 10 |
| | 1,3-butylene glycol | 20 | 20 | 20 | 20 |
| | methylparaben | 0.1 | 0.1 | 0.1 | 0.1 |
| Additive | 1% aqueous solution of sodium hydroxide | 2.6 | 2.6 | 2.6 | 2.6 |
| | 1.5% aqueous solution of carbomer *3 | 6.7 | 6.7 | 6.7 | 6.7 |
| Evaluation | W/O/W production efficiency | ◎ | ◎ | ◎ | ◎ |
| | Stability | ◎ | ◎ | ◎ | ◎ |

*1  RHEODOL TW-O120, manufactured by Kao Corporation, HLB: 15.0

*2  RHEODOL AO-15, manufactured by Kao Corporation, HLB: 3.7

*3  CARBOPOL 940, manufactured by Noveon Corporation

As is evident from Tables 11 and 12, even though the W/O/W emulsion compositions of examples 9 to 17 contained a polyhydric alcohol within the water phase and were prepared using a widely used conventional phase inversion emulsification sequence, they exhibited a high production efficiency for the W/O/W emulsion product and extremely favorable composition stability. In contrast, in the comparative examples 3 to 5, which did not contain the sugar fatty acid ester composition that represents an essential component of the present invention, absolutely no W/O/W emulsion product was generated.
[Table 13]

| | Table 13: W/O/W emulsion composition formulations (% by mass) and evaluation results | | | | | | |
|---|---|---|---|---|---|---|---|

| | Component | Example 18 | Example 19 | Example 20 | Comparative example 6 | Comparative example 7 | Comparative example 8 |
|---|---|---|---|---|---|---|---|
| Oil phase | T-iSt1 (hydroxyl value: 142) | 5 | - | - | - | - | - |
| | T-iSt3 (hydroxyl value: 57) | - | 5 | - | - | - | - |
| | T-iSt4 (hydroxyl value: 42) | - | - | 5 | - | - | - |
| | T-iSt5 (hydroxyl value: 350) | - | - | - | 5 | - | - |
| | T-iSt6 (hydroxyl value: 238) | - | - | - | - | 5 | - |
| | T-iSt7 (hydroxyl value: 5) | - | - | - | - | - | 5 |
| | Polyoxyethylene (20) sorbitan monooleate *1 | 0.7 | 0.7 | 0.7 | 0.7 | 0.7 | 0.7 |
| | Sorbitan sesquioleate *2 | 0.3 | 0.3 | 0.3 | 0.3 | 0.3 | 0.3 |
| | Cetyl 2-ethylhexanoate | 5 | 5 | 5 | 5 | 5 | 5 |
| Water phase | Pure water | 64.6 | 64.6 | 64.6 | 64.6 | 64.6 | 64.6 |
| | Glycerol | 5 | 5 | 5 | 5 | 5 | 5 |
| | 1,3-butylene glycol | 10 | 10 | 10 | 10 | 10 | 10 |
| | methylparaben | 0.1 | 0.1 | 0.1 | 0.1 | 0.1 | 0.1 |

| Additive | 1% aqueous solution of sodium hydroxide | 2.6 | 2.6 | 2.6 | 2.6 | 2.6 | 2.6 |
|---|---|---|---|---|---|---|---|
| | 1.5% aqueous solution of carbomer *3 | 6.7 | 6.7 | 6.7 | 6.7 | 6.7 | 6.7 |
| Evaluation | W/O/W production efficiency | ◎ | ◎ | ◎ | × | × | △ |
| | Stability | ◎ | ◎ | ○ | × | × | × |

*1  RHEODOL TW-O120, manufactured by Kao Corporation, HLB: 15.0

*2  RHEODOL AO-15, manufactured by Kao Corporation, HLB: 3.7

*3  CARBOPOL 940, manufactured by Noveon Corporation

[Table 14]

| | Component | Example 21 | Example 22 | Example 23 | Comparative example 9 | Comparative example 10 | Comparative example 11 | Comparative example 12 |
|---|---|---|---|---|---|---|---|---|
| | Table 14: W/O/W emulsion composition formulations (% by mass) and evaluation results | | | | | | | |
| Oil phase | RA-iSt1 (hydroxyl value: 124) | 5 | - | - | - | - | - | - |
| | RA-iSt3 (hydroxyl value: 21) | - | 5 | - | - | - | - | - |
| | LT-iSt1 (hydroxyl value: 189) | - | - | 5 | - | - | - | - |
| | RA-iSt4 (hydroxyl value: 335) | - | - | - | 5 | - | - | - |
| | RA-iSt5 (hydroxyl value: 5) | - | - | - | - | 5 | - | - |
| | LT-iSt3 (hydroxyl value: 301) | - | - | - | - | - | 5 | - |
| | LT-iSt4 (hydroxyl value: 6) | - | - | - | - | - | - | 5 |
| | Polyoxyethylene (20) sorbitan monooleate *1 | 0.7 | 0.7 | 0.7 | 0.7 | 0.7 | 0.7 | 0.7 |
| | Sorbitan sesquioleate *2 | 0.3 | 0.3 | 0.3 | 0.3 | 0.3 | 0.3 | 0.3 |
| | Cetyl 2-ethylhexanoate | 5 | 5 | 5 | 5 | 5 | 5 | 5 |
| Water phase | Pure water | 64.6 | 64.6 | 64.6 | 64.6 | 64.6 | 64.6 | 64.6 |
| | Glycerol | 5 | 5 | 5 | 5 | 5 | 5 | 5 |
| | 1,3-butylene glycol | 10 | 10 | 10 | 10 | 10 | 10 | 10 |
| | methylparaben | 0.1 | 0.1 | 0.1 | 0.1 | 0.1 | 0.1 | 0.1 |
| Additive | 1% aqueous solution of sodium hydroxide | 2.6 | 2.6 | 2.6 | 2.6 | 2.6 | 2.6 | 2.6 |
| | 1.5% aqueous solution of carbomer *3 | 6.7 | 6.7 | 6.7 | 6.7 | 6.7 | 6.7 | 6.7 |
| Evaluation | W/O/W production efficiency | ◎ | ○ | ◎ | △ | △ | × | △ |
| | Stability | ◎ | ◎ | ◎ | × | × | × | × |

*1 RHEODOL TW-O120, manufactured by Kao Corporation, HLB: 15.0

*2 RHEODOL AO-15, manufactured by Kao Corporation, HLB: 3.7

*3 CARBOPOL 940, manufactured by Noveon Corporation

As is evident from Tables 13 and 14, the W/O/W emulsion compositions of examples 18 to 23, each of which used a sugar fatty acid ester composition having a hydroxyl value within a range from 20 to 220, were prepared using a widely used conventional phase inversion emulsification sequence, and yet exhibited a high production efficiency for the W/O/W emulsion product and favorable composition stability. In contrast, in the comparative examples 6 to 12, which used a sugar fatty acid ester composition having a hydroxyl value outside the range from 20 to 220, the production efficiency for the W/O/W emulsion product was poor, and the stability of the composition was also unfavorable.

[Table 15]

| | Component | Example 24 | Example 25 | Example 26 | Example 27 | Example 28 | Example 29 | Example 30 |
|---|---|---|---|---|---|---|---|---|
| | Table 15: W/O/W emulsion composition formulations (% by mass) and evaluation results | | | | | | | |
| Oil phase | T-La (hydroxyl value: 36) | 5 | - | - | - | - | - | - |
| | T-Pa (hydroxyl value: 110) | - | 5 | - | - | - | - | - |
| | T-Ol (hydroxyl value: 95) | - | - | 5 | - | - | - | - |
| | T-HD (hydroxyl value: 100) | - | - | - | 5 | - | - | - |
| | RA-St (hydroxyl value: 130) | - | - | - | - | 5 | - | - |
| | RA-EH (hydroxyl value: 101) | - | - | - | - | - | 5 | - |
| | LT-DT (hydroxyl value: 59) | - | - | - | - | - | - | 5 |
| | Polyoxyethylene (20) sorbitan monooleate *1 | 0.7 | 0.7 | 0.7 | 0.7 | 0.7 | 0.7 | 0.7 |
| | Sorbitan sesquioleate *2 | 0.3 | 0.3 | 0.3 | 0.3 | 0.3 | 0.3 | 0.3 |
| | Cetyl 2-ethylhexanoate | 5 | 5 | 5 | 5 | 5 | 5 | 5 |
| Water phase | Pure water | 64.6 | 64.6 | 64.6 | 64.6 | 64.6 | 64.6 | 64.6 |
| | Glycerol | 5 | 5 | 5 | 5 | 5 | 5 | 5 |
| | 1,3-butylene glycol | 10 | 10 | 10 | 10 | 10 | 10 | 10 |
| | methylparaben | 0.1 | 0.1 | 0.1 | 0.1 | 0.1 | 0.1 | 0.1 |
| Additive | 1% aqueous solution of sodium hydroxide | 2.6 | 2.6 | 2.6 | 2.6 | 2.6 | 2.6 | 2.6 |
| | 1.5% aqueous solution of carbomer *3 | 6.7 | 6.7 | 6.7 | 6.7 | 6.7 | 6.7 | 6.7 |
| Evaluation | W/O/W production efficiency | ○ | ◎ | ◎ | ◎ | ◎ | ○ | ◎ |
| | Stability | ◎ | ◎ | ◎ | ◎ | ◎ | ◎ | ◎ |

*1  RHEODOL TW-O120, manufactured by Kao Corporation, HLB: 15.0

*2  RHEODOL AO-15, manufactured by Kao Corporation, HLB: 3.7

*3  CARBOPOL 940, manufactured by Noveon Corporation

As is evident from Table 15, when sugar fatty acid ester compositions obtained by performing esterifications of a variety of different fatty acids were used to prepare W/O/W emulsion compositions, even though preparation was conducted using a widely used conventional phase inversion emulsification sequence, the production efficiency for the W/O/W emulsion product was excellent and the stability was also favorable.

[Table 16]

| Table 16:  W/O/W emulsion composition formulations (% by mass) and evaluation results | | | | | | | |
|---|---|---|---|---|---|---|---|
| | Component | Example 31 | Example 32 | Example 33 | Example 34 | Example 35 | Example 36 |
| Oil phase | T-iSt2 (hydroxyl value: 94) | 5 | 5 | 5 | 5 | 5 | 5 |
| | Decaglyceryl monooleate  *4 | 1 | - | - | - | - | - |
| | Polyoxyethylene hydrogenated castor oil (20 E.O.)  *5 | - | 1 | - | - | - | - |
| | Polyoxyethylene sorbitol tetraoleate (60 E.O.)  *6 | - | - | 1 | - | - | - |
| | Polyoxyethylene (5) lauryl ether *7 | - | - | - | 1 | - | - |
| | Polyoxyethylene (13) cetyl ether *8 | - | - | - | - | 1 | - |
| | Polyethylene glycol monostearate (40 E.O.)  *9 | - | - | - | - | - | 1 |
| | Cetyl 2-ethylhexanoate | 5 | 5 | 5 | 5 | 5 | 5 |
| Water phase | Pure water | 64.6 | 64.6 | 64.6 | 64.6 | 64.6 | 64.6 |
| | Glycerol | 5 | 5 | 5 | 5 | 5 | 5 |
| | 1,3-butylene glycol | 10 | 10 | 10 | 10 | 10 | 10 |
| | methylparaben | 0.1 | 0.1 | 0.1 | 0.1 | 0.1 | 0.1 |

| Additive | 1% aqueous solution of sodium hydroxide | 2.6 | 2.6 | 2.6 | 2.6 | 2.6 | 2.6 |
|---|---|---|---|---|---|---|---|
| | 1.5% aqueous solution of carbomer *3 | 6.7 | 6.7 | 6.7 | 6.7 | 6.7 | 6.7 |
| Evaluation | W/O/W production efficiency | ○ | ◎ | ◎ | ◎ | ◎ | ◎ |
| | Stability | ◎ | ◎ | ◎ | ◎ | ◎ | ◎ |

*3  CARBOPOL 940, manufactured by Noveon Corporation

*4  SALACOS PG-180, manufactured by The Nisshin OilliO Group, Ltd., HLB: 14.8

*5  NIKKOL HCO-20, manufactured by Nikko Chemicals Co., Ltd., HLB: 10.5

*6  RHEODOL 460, manufactured by Kao Corporation, HLB: 13.8

*7  EMULGEN 106, manufactured by Kao Corporation, HLB: 10.5

*8  EMULGEN 220, manufactured by Kao Corporation, HLB: 14.2

*9  NIKKOL MYS-40, manufactured by Nikko Chemicals Co., Ltd., HLB: 17.5

As is evident from Table 16, when a variety of different nonionic surfactants having an HLB value of not less than 7 were used to prepare W/O/W emulsion compositions, even though preparation was conducted using a widely used conventional phase inversion emulsification sequence, the production efficiency for the W/O/W emulsion product was excellent and the stability was also favorable.
[Table 17]

Table 17: Example 37  W/O/W milky lotions

| Component | Name of component | Blend amount (% by mass) |
|---|---|---|
| 1 | T-iSt2 (hydroxyl value: 94) | 2.0 |
| 2 | Polyoxyethylene (20) sorbitan monostearate (HLB: 15.0) | 0.7 |
| 3 | Sorbitan sesquioleate | 0.3 |
| 4 | Glyceryl tri-2-ethylhexanoate | 8.0 |
| 5 | 2-ethylhexyl palmitate | 2.0 |
| 6 | Liquid paraffin | 10.0 |
| 7 | Dimethylpolysiloxane | 0.5 |
| 8 | Cetanol | 0.5 |
| 9 | Glycerol | 5.0 |
| 10 | 1,3-butylene glycol | 15.0 |
| 11 | Methylparaben | 0.1 |
| 12 | Ion-exchanged water | 39.3 |
| 13 | 1% aqueous solution of sodium hydroxide | 2.6 |
| 14 | 1% aqueous solution of carboxyvinyl polymer | 10.0 |
| 15 | 1.5% aqueous solution of hydroxypropyl methyl cellulose | 2.0 |
| 16 | 1% aqueous solution of xanthan gum | 1.0 |
| 17 | 1% solution of sodium hyaluronate | 1.0 |
|  | Total | 100 |

The components 1 to 8 and the components 9 to 12 of example 37 were combined in separate containers, each container was heated to 70˚C to dissolve the contents, and the container containing the components 1 to 8 was stirred with a disper while the components 9 to 12 were added gradually. The resulting mixture was then cooled to 40˚C or lower, the components 13 and 14 were added, the components 15 to 17 were then added, and the resulting mixture was stirred until uniform, yielding W/O/W milky lotions. Although these W/O/W milky lotions were prepared using a widely used conventional phase inversion emulsification sequence, the production efficiency for the W/O/W emulsion product was excellent and the stability was also favorable.
[Table 18]

| Table 18: Example 38 W/O/W cream | | |
|---|---|---|
| Component | Name of component | Blend amount (% by mass) |
| 1 | T-iSt2 (hydroxyl value: 94) | 4.0 |
| 2 | Polyoxyethylene (20) sorbitan monooleate (HLB: 15.0) | 1.6 |
| 3 | Sorbitan sesquistearate | 0.4 |
| 4 | Glyceryl tri-2-ethylhexanoate | 5.0 |
| 5 | Neopentyl glycol dicaprate | 15.0 |
| 6 | Squalane | 10.0 |
| 7 | Dimethylpolysiloxane | 1.0 |
| 8 | Batyl alcohol | 2.0 |
| 9 | Glycerol | 5.0 |
| 10 | 1,3-butylene glycol | 10.0 |
| 11 | Methylparaben | 0.1 |
| 12 | Ion-exchanged water | 25.0 |
| 13 | 1% aqueous solution of sodium hydroxide | 3.9 |
| 14 | 1% aqueous solution of carboxyvinyl polymer | 15.0 |
| 15 | 1.5% aqueous solution of hydroxyethyl cellulose | 2.0 |
| | Total | 100 |

The components 1 to 8 and the components 9 to 12 of example 38 were combined in separate containers, each container was heated to 70˚C to dissolve the contents, and the container containing the components 1 to 8 was stirred with a homomixer while the components 9 to 12 were added gradually. The resulting mixture was then cooled to 40˚C or lower, the components 13 and 14 were added, the component 15 was then added, and the resulting mixture was stirred until uniform, yielding a W/O/W cream. Although this W/O/W cream was prepared using a widely used conventional phase inversion emulsification sequence, the production efficiency for the W/O/W emulsion product was excellent and the stability was also favorable.

[Table 19]

| Table 19:  Example 39  W/O/W gel-like essence | | |
|---|---|---|
| Component | Name of component | Blend amount (% by mass) |
| 1 | RA-iSt2 (hydroxyl value: 53) | 1.0 |
| 2 | Polyoxyethylene (13) cetyl ether (HLB: 14.2) | 0.2 |
| 3 | Squalane | 2.0 |
| 4 | Pentaerythrityl tetra-2-ethylhexanoate | 2.0 |
| 5 | Dimethylpolysiloxane | 0.2 |
| 6 | Glycerol | 10.0 |
| 7 | 1,3-butylene glycol | 10.0 |
| 8 | Methylparaben | 0.2 |
| 9 | Ion-exchanged water | 42.9 |
| 10 | 1% aqueous solution of sodium hydroxide | 6.5 |
| 11 | 1% aqueous solution of carboxyvinyl polymer | 25.0 |
|  | Total | 100 |

The components 1 to 5 and the components 6 to 9 of example 39 were combined in separate containers, each container was heated to 70˚C to dissolve the contents, and the container containing the components 1 to 5 was stirred with a disper while the components 6 to 9 were added gradually. The resulting mixture was then cooled to 40˚C or lower, the components 10 and 11 were added, and the resulting mixture was stirred until uniform, yielding a W/O/W gel-like essence. Although this W/O/W gel-like essence was prepared using a widely used conventional phase inversion emulsification sequence, the production efficiency for the W/O/W emulsion product was excellent and the stability was also favorable.
[Table 20]

| Table 20: Example 40 W/O/W pack cosmetics preparation | | |
|---|---|---|
| Component | Name of component | Blend amount (% by mass) |
| 1 | T-iSt2 (hydroxyl value: 94) | 0.5 |
| 2 | Polyoxyethylene (20) hydrogenated castor oil (HLB: 10.5) | 0.2 |
| 3 | Squalane | 1.0 |
| 4 | Dimethylpolysiloxane | 0.1 |
| 5 | Glycerol | 2.0 |
| 6 | 1,3-butylene glycol | 4.0 |
| 7 | Methylparaben | 0.05 |
| 8 | Ion-exchanged water | 20.0 |
| 9 | Polyvinyl alcohol | 15.0 |
| 10 | Carboxyvinyl polymer | 0.25 |
| 11 | Potassium hydroxide | 0.08 |
| 12 | Methylparaben | 0.05 |
| 13 | Ion-exchanged water | 46.77 |
| 14 | Ethanol | 10.0 |
| | Total | 100 |

The components 1 to 4 and the components 5 to 8 of example 40 were combined in separate containers, each container was heated to 70°C to dissolve the contents, the container containing the components 1 to 4 was stirred with a disper while the components 5 to 8 were added gradually, and the resulting mixture was then cooled to 40°C or lower. The resulting W/O/W emulsion product was added to a separately prepared uniform solution containing the components 9 to 13, the component 14 was then added, and the resulting mixture was stirred until uniform, yielding a W/O/W pack cosmetics preparation. In the production of this W/O/W pack cosmetics preparation, the preparation of the W/O/W emulsion product was conducted using a widely used conventional phase inversion emulsification sequence, and the subsequent preparation sequence was no different from the methods used in obtaining conventional pack cosmetics preparations, meaning the product was obtained via a very simple process. In the case of this W/O/W pack cosmetics preparation, the production efficiency for the W/O/W emulsion product was excellent and the stability was also favorable.

Formulations listed below in Tables 21 to 24 were used to prepare W/O/W emulsion compositions using the same preparation method as that described above. The thus obtained W/O/W emulsion compositions were evaluated using the same evaluation methods and criteria as those described above, by observing the emulsion state of each composition immediately following preparation and then after storage at 40°C for one month, and using the results of the observations to evaluate the production efficiency and the stability of the W/O/W emulsion product.

In the case of examples 59 and 60, the water retention of the W/O/W emulsion composition was also evaluated using the evaluation method and evaluation criteria described below.

[Method and criteria for evaluating water retention of the W/O/W emulsion composition]

An approximately 3 g sample of the prepared W/O/W emulsion composition was placed on a weighing dish, and the weight of the sample was measured. Subsequently, the sample was stored for 15 hours under conditions including a temperature of 40°C and a humidity of 50%, the weight of the sample was re-measured, and the weight ratio of the weight following storage relative to the weight prior to testing was evaluated against the following criteria.
◎: the weight ratio of the weight following storage relative to the weight prior to testing was 25% or greater.

◯: the weight ratio of the weight following storage relative to the weight prior to testing was at least 20% but less than 25%.

×: the weight ratio of the weight following storage relative to the weight prior to testing was less than 20%.

[Table 21]

| Table 21: W/O/W emulsion composition formulations (% by mass) and evaluation results | | | | | | | |
|---|---|---|---|---|---|---|---|
| | Component | Example 41 | Example 42 | Example 43 | Example 44 | Example 45 | Example 46 |
| Oil phase | T-iSt2 (hydroxyl value: 94) | 5 | 5 | 5 | 5 | 5 | 5 |
| | Decaglyceryl monooleate *4 | 0.7 | - | - | - | - | - |
| | Diglyceryl monooleate *10 | 0.3 | - | - | - | - | - |
| | Polyoxyethylene hydrogenated castor oil (60 E.O.) *11 | - | 1 | - | - | - | - |
| | Polyoxyethylene hydrogenated castor oil (40 E.O.) *12 | - | - | 1 | - | - | - |
| | Polyoxyethylene hydrogenated castor oil (25 E.O.) *13 | - | - | - | 1 | - | - |
| | PEG-20 glyceryl isostearate *14 | - | - | - | - | 1 | - |
| | Polyoxyethylene behenyl ether (30 E.O.) *15 | - | - | - | - | - | 1 |
| | Liquid paraffin | 5 | 5 | 5 | 5 | 5 | 5 |
| Water phase | Pure water | 76.3 | 76.3 | 76.3 | 76.3 | 76.3 | 76.3 |
| | methylparaben | 0.1 | 0.1 | 0.1 | 0.1 | 0.1 | 0.1 |
| Additive | 1% aqueous solution of sodium hydroxide | 2.6 | 2.6 | 2.6 | 2.6 | 2.6 | 2.6 |
| | 1% aqueous solution of carbomer *3 | 10.0 | 10.0 | 10.0 | 10.0 | 10.0 | 10.0 |
| Evaluation | W/O/W production efficiency | ○ | ◎ | ◎ | ◎ | ○ | ○ |
| | Stability | ○ | ◎ | ◎ | ◎ | ◎ | ◎ |

*3  CARBOPOL 940, manufactured by Noveon Corporation

*4  SALACOS PG-180, manufactured by The Nisshin OilliO Group, Ltd., HLB: 14.8

*10  SALACOS DG-180, manufactured by The Nisshin OilliO Group, Ltd., HLB: 7.4

*11  EMANON CH-60(K), manufactured by Kao Corporation, HLB: 14.0

*12  EMANON CH-40, manufactured by Kao Corporation, HLB: 12.5

*13  EMANON CH-25, manufactured by Kao Corporation, HLB: 10.7

*14  SALACOS GE-118, manufactured by The Nisshin OilliO Group, Ltd., HLB: 13.0

*15  EMALEX BHA-30, manufactured by Nihon-Emulsion Co., Ltd., HLB: 14.0

As is evident from Table 21, when a variety of different nonionic surfactants having an HLB value of not less than 7 were used to prepare W/O/W emulsion compositions, even though preparation was conducted using a widely used conventional phase inversion emulsification sequence, the production efficiency for the W/O/W emulsion product was excellent and the stability was also favorable. Use of a polyoxyethylene hydrogenated castor oil yielded an emulsion product with particularly favorable levels of production efficiency and stability for the W/O/W emulsion product.
[Table 22]

Table 22: W/O/W emulsion composition formulations (% by mass) and evaluation results

| | Component | Example 47 | Example 48 | Example 49 | Example 50 | Example 51 | Example 52 | Example 53 |
|---|---|---|---|---|---|---|---|---|
| Oil phase | T-iSt2 (hydroxyl value: 94) | 4 | 3 | 5 | 6 | 5 | 5 | 3 |
| | Polyoxyethylene hydrogenated castor oil (60 E.O.) *11 | 1 | 1 | 1 | 2 | 1 | 1 | 1 |
| | Liquid paraffin | 6 | 7 | 10 | 14 | 5 | 5 | 7 |
| Water phase | Pure water | 76.3 | 76.3 | 71.3 | 65.3 | 66.3 | 66.3 | 66.3 |
| | Glycerol | - | - | - | - | 5 | - | 5 |
| | 1,3-butylene glycol | - | - | - | - | 5 | 10 | 5 |
| | methylparaben | 0.1 | 0.1 | 0.1 | 0.1 | 0.1 | 0.1 | 0.1 |
| Additive | 1% aqueous solution of sodium hydroxide | 2.6 | 2.6 | 2.6 | 2.6 | 2.6 | 2.6 | 2.6 |
| | 1% aqueous solution of carbomer *3 | 10.0 | 10.0 | 10.0 | 10.0 | 10.0 | 10.0 | 10.0 |
| Evaluation | W/O/W production efficiency | ◎ | ◎ | ◎ | ◎ | ◎ | ◎ | ◎ |
| | Stability | ◎ | ◎ | ◎ | ◎ | ◎ | ◎ | ◎ |

*3 CARBOPOL 940, manufactured by Noveon Corporation

*11 EMANON CH-60(K), manufactured by Kao Corporation, HLB: 14.0

As is evident from Table 22, in examples 47 and 48, in which the blend ratio between the sugar fatty acid ester and the nonionic surfactant having an HLB value of not less than 7 was varied, even though preparation was conducted using a widely used conventional phase inversion emulsification sequence, the production efficiency for the W/O/W emulsion product was excellent and the stability was also favorable. Even in examples 49 and 50, in which the blend amount of the combined oil phase was increased, the production efficiency for the W/O/W emulsion product was excellent and the stability was also favorable.
[Table 23]

| Table 23: W/O/W emulsion composition formulations (% by mass) and evaluation results | | | | | | |
|---|---|---|---|---|---|---|
| | Component | Example 54 | Example 55 | Example 56 | Example 57 | Example 58 |
| Oil phase | T-iSt2 (hydroxyl value: 94) | 5 | 3 | 5 | 6 | 5 |
| | Polyoxyethylene hydrogenated castor oil (60 E.O.) *11 | 1 | 1 | 1 | 1 | 1 |
| | Squalane | 5 | 5 | 5 | - | - |
| | Neopentyl glycol dicaprate *16 | - | - | - | 5 | - |
| | Glyceryl tri-2-ethylhexanoate *17 | - | - | - | - | 5 |
| Water phase | Pure water | 76.3 | 73.3 | 66.3 | 75.3 | 77.3 |
| | 1,3-butylene glycol | - | 5 | 10 | - | - |
| | methylparaben | 0.1 | 0.1 | 0.1 | 0.1 | 0.1 |

| Additive | 1% aqueous solution of sodium hydroxide | 2.6 | 2.6 | 2.6 | 2.6 | 2.6 |
|---|---|---|---|---|---|---|
| | 1% aqueous solution of carbomer *3 | 10.0 | 10.0 | 10.0 | 10.0 | 10.0 |
| Evaluation | W/O/W production efficiency | ◎ | ◎ | ◎ | ◎ | ◎ |
| | Stability | ◎ | ◎ | ◎ | ◎ | ◎ |

*3  CARBOPOL 940, manufactured by Noveon Corporation

*11  EMANON CH-60(K), manufactured by Kao Corporation, HLB: 14.0

*16  ESTEMOL N-01, manufactured by The Nisshin OilliO Group, Ltd.

*17  T.I.O, manufactured by The Nisshin OilliO Group, Ltd.

As is evident from Table 23, when a variety of different hydrocarbon oils and ester oils were combined with the sugar fatty acid ester composition in the oil phase during preparation of the W/O/W emulsion composition, even though the preparation was conducted using a widely used conventional phase inversion emulsification sequence, the production efficiency for the W/O/W emulsion product was excellent and the stability was also favorable.

[Table 24]

| Table 24: W/O/W emulsion composition formulations (% by mass) and evaluation results | | | |
|---|---|---|---|
| | Component | Example 59 | Example 60 |
| Oil phase | T-iSt2 (hydroxyl value: 94) | 5 | 5 |
| | Polyoxyethylene hydrogenated castor oil (60 E.O.) *11 | 1 | - |
| | Decaglyceryl monooleate *4 | - | 0.7 |
| | Diglyceryl monooleate *10 | - | 0.3 |
| | Liquid paraffin | 5 | 5 |
| Water phase | Pure water | 76.3 | 76.3 |
| | methylparaben | 0.1 | 0.1 |
| Additive | 1% aqueous solution of sodium hydroxide | 2.6 | 2.6 |
| | 1% aqueous solution of carbomer *3 | 10.0 | 10.0 |
| Evaluation | W/O/W production efficiency | ◎ | ○ |
| | Water retention | ◎ | ○ |
| | Stability | ◎ | ◎ |

*3  CARBOPOL 940, manufactured by Noveon Corporation

*4  SALACOS PG-180, manufactured by The Nisshin OilliO Group, Ltd., HLB: 14.8

*10  SALACOS DG-180, manufactured by The Nisshin OilliO Group, Ltd., HLB: 7.4

*11  EMANON CH-60(K), manufactured by Kao Corporation, HLB: 14.0

As is evident from Table 24, even though the preparation of examples 59 and 60 was conducted using a widely used conventional phase inversion emulsification sequence, the production efficiency for the W/O/W emulsion product was excellent and the stability was also favorable. Further, in the case of example 59, which used a polyoxyethylene hydrogenated castor oil as the nonionic surfactant, the water retention was also particularly favorable.

INDUSTRIAL APPLICABILITY

The present invention is able to provide a W/O/W emulsion composition that can be prepared using a widely used and simple conventional phase inversion emulsification method, and for which the emulsion stability is high and the production efficiency of the W/O/W emulsion product is excellent.

**Claims**

1.  A W/O/W emulsion composition comprising: 0.001 to 60% by mass of a component (A) described below and 0.001 to 10% by mass of a component (B) described below; wherein, a mass ratio between said component (A) and said component (B) is within a range from 1:0.01 to 1:1.4.
    Component (A): a sugar fatty acid ester composition obtained by esterifying a sugar and a fatty acid of 8 to 28 carbon atoms, in which a hydroxyl value of said sugar fatty acid ester composition is within a range from 20 to 220.
    Component (B): a nonionic surfactant having an HLB value of not less than 7.

2.  The W/O/W emulsion composition according to claim 1, wherein a constituent sugar of said sugar fatty acid ester composition of said component (A) is one or more sugars selected from the group consisting of inositol, trehalose, lactitol, maltitol, raffinose, mannitol, xylitol and erythritol.

3.  The W/O/W emulsion composition according to claim 1, wherein said constituent sugar of said sugar fatty acid ester composition of said component (A) is trehalose.

4.  The W/O/W emulsion composition according to any one of claims 1 to 3, wherein said constituent fatty acid of 8 to 28 carbon atoms of said sugar fatty acid ester composition of said component (A) is one or more compounds selected from the group consisting of octanoic acid, decanoic acid, lauric acid, myristic acid, palmitic acid, stearic acid, arachidonic acid, palmitoleic acid, oleic acid, linoleic acid, linolenic acid, behenic acid, erucic acid, 2-ethylhexanoic acid, 2-hexyldecanoic acid, 2-heptylundecylenic acid, isostearic acid, 2-octyldodecanoic acid and 2-dodecyltetradecanoic acid.

5.  The W/O/W emulsion composition according to any one of claims 1 to 3, wherein said constituent fatty acid of 8 to 28 carbon atoms of said sugar fatty acid ester composition of said component (A) is isostearic acid.

6.  The W/O/W emulsion composition according to any one of claims 1 to 5, wherein said component (B) is a nonionic surfactant having an HLB value of not less than 7 that contains a polyoxyethylene group.

7.  The W/O/W emulsion composition according to any one of claims 1 to 6, further comprising: 0.1 to 45% by mass of a polyhydric alcohol as a component (C).

8.  The W/O/W emulsion composition according to claim 7, wherein said component (C) is one or more polyhydric alcohols selected from the group consisting of glycerol, diglycerol, triglycerol, polyethylene glycol, propylene glycol, 1,3-butylene glycol, 3-methyl-1,3-butanediol, 1,2-pentanediol, 1,2-hexanediol and hexylene glycol.

9.  The W/O/W emulsion composition according to any one of claims 1 to 8, wherein said W/O/W emulsion composition is a cosmetic preparation.

10. The W/O/W emulsion composition according to claim 9, wherein said cosmetic preparation is milky lotions, a cream, an essence, a lotion, an ointment or a pack.

11. A cosmetic preparation comprising: a W/O/W emulsion composition according to any one of claims 1 to 8.

12. The cosmetic preparation according to claim 11, wherein said cosmetic preparation is milky lotions, a cream, an essence, a lotion, an ointment or a pack.

13. A method of producing a W/O/W emulsion composition, said method comprising:

    adding a water phase containing water to an oil phase, and
    performing a phase inversion emulsification, wherein
    said oil phase comprises 0.001 to 60% by mass of a component (A) described below and 0.001 to 10% by mass of a component (B) described below, and
    a mass ratio between said component (A) and said component (B) is within a range from 1:0.01 to 1:1.4.
    Component (A): a sugar fatty acid ester composition obtained by esterifying a sugar and a fatty acid of 8 to 28 carbon atoms, in which a hydroxyl value of said sugar fatty acid ester composition is within a range from 20 to 220.
    Component (B): a nonionic surfactant having an HLB value of not less than 7.

## INTERNATIONAL SEARCH REPORT

International application No.

PCT/JP2008/061831

A. CLASSIFICATION OF SUBJECT MATTER
*B01J13/00*(2006.01)i, *A61K8/34*(2006.01)i, *A61K8/37*(2006.01)i, *A61K8/60*
(2006.01)i, *A61K8/84*(2006.01)i, *A61Q19/00*(2006.01)i

According to International Patent Classification (IPC) or to both national classification and IPC

B. FIELDS SEARCHED

Minimum documentation searched (classification system followed by classification symbols)
B01J13/00, A61K8/00, A61K9/00, A61Q19/00, B01F17/00

Documentation searched other than minimum documentation to the extent that such documents are included in the fields searched
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | WO 2007/063902 A1 (The Nisshin Oillio Group, Ltd.), 07 June, 2007 (07.06.07), Full text (Family: none) | 1-13 |
| A | JP 5-168893 A (Kanebo, Ltd.), 02 July, 1993 (02.07.93), Full text (Family: none) | 1-13 |
| A | JP 6-239717 A (Kanebo, Ltd.), 30 August, 1994 (30.08.94), Full text (Family: none) | 1-13 |

☒ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06 October, 2008 (06.10.08) | 14 October, 2008 (14.10.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

| | International application No. |
|---|---|
| | PCT/JP2008/061831 |

C (Continuation). DOCUMENTS CONSIDERED TO BE RELEVANT

| Category* | Citation of document, with indication, where appropriate, of the relevant passages | Relevant to claim No. |
|---|---|---|
| A | WO 2006/003992 A1 (The Nisshin Oillio Group, Ltd.), 12 January, 2006 (12.01.06), Full text & US 2007/0110702 A1 & EP 1782789 A1 & KR 10-2007-0029759 A & CN 1980628 A | 1-13 |
| A | WO 2004/100917 A1 (The Nisshin Oillio Group, Ltd.), 25 November, 2004 (25.11.04), Full text & US 2006/0067902 A1 & EP 1623695 A1 & KR 10-2006-0009339 A & CN 1787799 A | 1-13 |
| A | JP 2004-339094 A (The Nisshin Oillio Group, Ltd.), 02 December, 2004 (02.12.04), Full text (Family: none) | 1-13 |
| A | JP 11-209231 A (Dai-Ichi Kogyo Seiyaku Co., Ltd.), 03 August, 1999 (03.08.99), Full text (Family: none) | 1-13 |
| A | JP 2003-275573 A (Sunstar Inc.), 30 September, 2003 (30.09.03), Full text (Family: none) | 1-13 |
| A | JP 2006-281182 A (Sunstar Inc.), 19 October, 2006 (19.10.06), Full text (Family: none) | 1-13 |

Form PCT/ISA/210 (continuation of second sheet) (April 2007)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007174456 A
- JP HEI1133391 B
- JP 2004307414 A
- JP 2005132794 A

**Non-patent literature cited in the description**

- Determination of Sucrose Fatty Acid Ester by High-performance Liquid Chromatography. *J. Oleo Sci.,* 2001, vol. 50 (4